# EUROPEAN PATENT SPECIFICATION

(11) **EP 2 121 031 B1**
(45) Date of publication and mention of the grant of the patent: **16.10.2013**
(21) Application number: 08726761.3
(22) Date of filing: 12.03.2008
(51) Int. Cl.: A61K 47/48, A61P 37/08

(54) **OLIGOMER-ANTIHISTAMINE CONJUGATES**
OLIGOMER-ANTIHISTAMIN-KONJUGATE
CONJUGUÉS D'OLIGOMÈRE ANTIHISTAMINIQUE

(30) Priority: 12.03.2007 US 906416 P; 13.11.2007 US 2970; 20.11.2007 US 3808
(43) Date of publication of application: 25.11.2009
(60) Divisional application: 12171683.1
(73) Proprietor: Nektar Therapeutics, San Francisco CA 94158 (US)
(72) Inventor: RIGGS-SAUTHIER, Jennifer, Huntsville, AL 35802 (US); ZHANG, Wen, Madison, AL 35758 (US); VIEGAS, Tacey, X., Madison, AL 35758 (US); BENTLEY, Michael, D., Huntsville, AL 35801 (US)
(74) Representative: Baldock, Sharon Claire
(86) International application number: PCT/US2008/003288
(87) International publication number: WO 2008/112257

(56) References cited:
- WO-A-99/63999
- WO-A-2005/058367
- US-A1- 2006 182 692

## Description

### FIELD OF THE INVENTION

This invention provides (among other things) a chemically modified antihistamine, diphenhydramine, that possess a certain advantages over the antihistamine lacking the chemical modification. The chemically modified antihistamine described herein relates to and/or has application(s) in (among others) the fields of drug discovery, pharmacotherapy, physiology, organic chemistry and polymer chemistry.

### BACKGROUND OF THE INVENTION

Antihistamines are antagonists of the histamine- 1 (H₁) receptor. Also known as "H₁] receptor blockers," these agents cause a reduction in smooth muscle contraction. In addition, H₁ receptor blockers can serve to neutralize the effects of the substantial release of histamine associated with hypersensitivity reactions. In humans, such hypersensitivity reactions include allergic responses some have to pollen, bee stings, certain foods, and so forth.

Diphenhydramine and hydroxyzine represent members of the "first generation" of antihistamines and remain as two of the most effective antihistamines commercially available. These and other first generation antihistamines are administered when an allergic reaction requires immediate and effective reversal of histamine release. Despite their demonstrated efficacy, many first generation antihistamines do not represent the first drug of choice in the treatment of patients suffering from common allergies. Clinicians and patients do not turn to these antihistamines to address routine allergic responses due to their side effects. Such side effects include significant drowsiness, as well as the possibilities of ataxia, dry mouth, flushed skin, irregular hear rhythm, blurred vision, photophobia, pupil dilation, urinary retention, constipation, difficulty concentrating, short-term memory loss, hallucinations, confusion, erectile dysfunction, and delirium.

Relatively more recent antihistamines (such as loratadine, astemizole, and terfenadine) that have become available were believed to offer the same efficacy of the first generation antihistamines, but without drowsiness. Experience has shown, however, that these newer antihistamines lack the effectiveness of their first generation counterparts, or that if given at higher doses to provided the desired effectiveness, also result in the side effect of drowsiness.

It would be advantageous, therefore, to provide antihistamines that have the same antihistaminic activity as first generations antihistamines but lack their drowsiness-inducing side effect.

The present invention seeks to address these and other needs in the art by providing (among other things) a conjugate of a water-soluble and non-peptidic oligomer and the antihistamine diphenhydramine.

### SUMMARY OF THE INVENTION

In one or more embodiments of the invention, a compound is provided, the compound comprising a residue of an antihistamine covalently attached via a stable or degradable linkage to a water-soluble and non-peptidic oligomer. Specifically the invention relates to compound having the following structure: 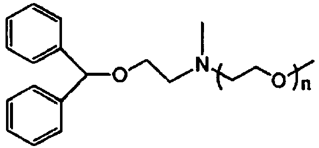 wherein:
n is an integer from 1 to 30.

### BRIEF DESCRIPTION OF THE FIGURES

FIG. 1 is a graph showing the concentration of conjugates in rat plasma of hydroxyzine and various hydroxyzine conjugates.

**FIG. 2** is a graph showing free (unbound) hydroxyzine in plasma in rats of orally dosed hydroxyzine and various hydroxyzine conjugates.

**FIG. 3** is a graph showing free (unbound) cetirizine in plasma in rats of orally dosed hydroxyzine and various hydroxyzine conjugates.

**FIG. 4** is a graph showing the rates of in vitro metabolism in rat liver microsomes of hydroxyzine and various hydroxyzine conjugates.

**FIG. 5** is a graph showing the rates of production of the metabolite cetirizine formed by liver microsomes following administration of hydroxyzine and various hydroxyzine conjugates.

**FIG. 6** is a graph showing the binding of PEG conjugates of diphenhydramine to the human H1 histamine receptor [wherein "PEG(5)-N-DPH" is mPEG(5)-N-Diphenhydramine; "PEG(6)-N-DPH" is mPEG(6)-N-Diphenhydramine; and "PEG(7)-N-DPH" is mPEG(7)-N-Diphenhydramine].

**FIG. 7** is a graph showing the binding of PEG conjugates of diphenhydramine to the human H1 histamine receptor [wherein "PEG-6-NH-DPH" is mPEG(6)-NH-Diphenhydramine and "PEG-7-N-DPH" is mPEG(7)-N-Diphenhydramine].

**FIG. 8** is a graph showing the effect of PEG size on binding of PEG-diphenhydramine conjugates to the human H1 histamine receptor [wherein "DPH" is diphenhydramine; "PEG-3-DPH" is mPEG(3)-N-Diphenhydramine; "PEG-4-DPH" is mPEG(4)-N-Diphenhydramine; "PEG-5-DPH" is mPEG(5)-N-Diphenhydramine; "PEG-6-DPH" is mPEG(6)-N-Diphenhydramine; and "PEG-7-DPH" is mPEG(7)-N-Diphenhydramine].

**FIG. 9** is a graph showing plasma concentration-time profiles of DPH and PEG-N-DPH conjugates following IV administration in rats.

**FIG. 10** is a graph showing plasma concentration-time profiles of DPH and PEG-N-DPH conjugates following oral administration in rats.

### DETAILED DESCRIPTION OF THE INVENTION

As used in this specification, the singular forms "a," "an," and "the" include plural referents unless the context clearly dictates otherwise.

In describing and claiming the present invention, the following terminology will be used in accordance with the definitions described below.

"Water soluble, non-peptidic oligomer" indicates an oligomer that is at least 35% (by weight) soluble, preferably greater than 70% (by weight), and more preferably greater than 95% (by weight) soluble, in water at room temperature. Typically, an unfiltered aqueous preparation of a "water-soluble" oligomer transmits at least 75%, more preferably at least 95%, of the amount of light transmitted by the same solution after filtering. It is most preferred, however, that the water-soluble oligomer is at least 95% (by weight) soluble in water or completely soluble in water. With respect to being "non-peptidic," an oligomer is non-peptidic when it has less than 35% (by weight) of amino acid residues.

The terms "monomer," "monomeric subunit" and "monomeric unit" are used interchangeably herein and refer to one of the basic structural units of a polymer or oligomer. In the case of a homo-oligomer, a single repeating structural unit forms the oligomer. In the case of a co-oligomer, two or more structural units are repeated -- either in a pattern or randomly -- to form the oligomer. Preferred oligomers used in connection with present the invention are homo-oligomers. The water-soluble, non-peptidic oligomer typically comprises one or more monomers serially attached to form a chain of monomers. The oligomer can be formed from a single monomer type (i.e., is homo-oligomeric) or two or three monomer types (i.e., is co-oligomeric).

An "oligomer" is a molecule possessing from about 2 to about 50 monomers, preferably from about 2 to about 30 monomers. The architecture of an oligomer can vary. Specific oligomers for use in the invention include those having a variety of geometries such as linear, branched, or forked, to be described in greater detail below.

"PEG" or "polyethylene glycol," as used herein, is meant to encompass any water-soluble poly(ethylene oxide). Unless otherwise indicated, a "PEG oligomer" (also called an oligoethylene glycol) is one in which substantially all (and more preferably all) monomeric subunits are ethylene oxide subunits. The oligomer may, however, contain distinct end capping moieties or functional groups, e.g., for conjugation. Typically, PEG oligomers for use in the present invention will comprise one of the two following structures: "-(CH₂CH₂O)ₙ-" or "-(CH₂CH₂O)ₙ₋₁CH₂CH₂-," depending upon whether the terminal oxygen(s) has been displaced, e.g., during a synthetic transformation. For PEG oligomers, "n" varies from about 2 to 50, preferably from about 2 to about 30, and the terminal groups and architecture of the overall PEG can vary. When PEG further comprises a functional group, A, for linking to, e.g., a small molecule drug, the functional group when covalently attached to a PEG oligomer does not result in formation of (i) an oxygen-oxygen bond (-O-O-, a peroxide linkage), or (ii) a nitrogen-oxygen bond (N-O, O-N).

An "end capping group" is generally a non-reactive carbon-containing group attached to a terminal oxygen of a PEG oligomer. Exemplary end capping groups comprise a C₁₋₅ alkyl group, such as methyl, ethyl and benzyl), as well as aryl, heteroaryl, cyclo, heterocyclo, and the like. For the purposes of the present invention, the preferred capping groups have relatively low molecular weights such as methyl or ethyl. The end-capping group can also comprise a detectable label. Such labels include, without limitation, fluorescers, chemiluminescers, moieties used in enzyme labeling, colorimetric labels (e.g., dyes), metal ions, and radioactive moieties.

"Branched", in reference to the geometry or overall structure of an oligomer, refers to an oligomer having two or more polymers representing distinct "arms" that extend from a branch point.

"Forked" in reference to the geometry or overall structure of an oligomer, refers to an oligomer having two or more functional groups (typically through one or more atoms) extending from a branch point.

A "branch point" refers to a bifurcation point comprising one or more atoms at which an oligomer branches or forks from a linear structure into one or more additional arms.

The term "reactive" or "activated" refers to a functional group that reacts readily or at a practical rate under conventional conditions of organic synthesis. This is in contrast to those groups that either do not react or require strong catalysts or impractical reaction conditions in order to react (i.e., a "nonreactive" or "inert" group).

"Not readily reactive," with reference to a functional group present on a molecule in a reaction mixture, indicates that the group remains largely intact under conditions that are effective to produce a desired reaction in the reaction mixture.

A "protecting group" is a moiety that prevents or blocks reaction of a particular chemically reactive functional group in a molecule under certain reaction conditions. The protecting group will vary depending upon the type of chemically reactive group being protected as well as the reaction conditions to be employed and the presence of additional reactive or protecting groups in the molecule. Functional groups which may be protected include, by way of example, carboxylic acid groups, amino groups, hydroxyl groups, thiol groups, carbonyl groups and the like. Representative protecting groups for carboxylic acids include esters (such as a p-methoxybenzyl ester), amides and hydrazides; for amino groups, carbamates (such as tert-butoxycarbonyl) and amides; for hydroxyl groups, ethers and esters; for thiol groups, thioethers and thioesters; for carbonyl groups, acetals and ketals; and the like. Such protecting groups are well-known to those skilled in the art and are described, for example, in T.W. Greene and G.M. Wuts, Protecting Groups in Organic Synthesis, Third Edition, Wiley, New York, 1999, and references cited therein.

A functional group in "protected form" refers to a functional group bearing a protecting group. As used herein, the term "functional group" or any synonym thereof encompasses protected forms thereof.

A "physiologically cleavable" or "hydrolyzable" or "degradable" bond is a relatively labile bond that reacts with water (i.e., is hydrolyzed) under ordinary physiological conditions. The tendency of a bond to hydrolyze in water under ordinary physiological conditions will depend not only on the general type of linkage connecting two central atoms but also on the substituents attached to these central atoms. Such bonds are generally recognizable by those of ordinary skill in the art. Appropriate hydrolytically unstable or weak linkages include but are not limited to carboxylate ester, phosphate ester, anhydrides, acetals, ketals, acyloxyalkyl ether, imines, orthoesters, peptides, oligonucleotides, thioesters, and carbonates.

An "enzymatically degradable linkage" means a linkage that is subject to degradation by one or more enzymes under ordinary physiological conditions.

A "stable" linkage or bond refers to a chemical moiety or bond, typically a covalent bond, that is substantially stable in water, that is to say, does not undergo hydrolysis under ordinary physiological conditions to any appreciable extent over an extended period of time. Examples of hydrolytically stable linkages include but are not limited to the following: carbon-carbon bonds (e.g., in aliphatic chains), ethers, amides, urethanes, amines, and the like. Generally, a stable linkage is one that exhibits a rate of hydrolysis of less than about 1-2% per day under ordinary physiological conditions. Hydrolysis rates of representative chemical bonds can be found in most standard chemistry textbooks.

In the context of describing the consistency of oligomers in a given composition, "substantially" or "essentially" means nearly totally or completely, for instance, 95% or greater, more preferably 97% or greater, still more preferably 98% or greater, even more preferably 99% or greater, yet still more preferably 99.9% or greater, with 99.99% or greater being most preferred of some given quantity.

"Monodisperse" refers to an oligomer composition wherein substantially all of the oligomers in the composition have a well-defined, single molecular weight and defined number of monomers, as determined by chromatography or mass spectrometry. Monodisperse oligomer compositions are in one sense pure, that is, substantially comprising molecules having a single and definable number of monomers rather than several different numbers of monomers (i.e., an oligomer composition having three or more different oligomer sizes). A monodisperse oligomer composition possesses a MW/Mn value of 1.0005 or less, and more preferably, a MW/Mn value of 1.0000. By extension, a composition comprised of monodisperse conjugates means that substantially all oligomers of all conjugates in the composition have a single and definable number (as a whole number) of monomers rather than a distribution and would possess a MW/Mn value of 1.0005, and more preferably, a MW/Mn value of 1.0000 if the oligomer were not attached to the residue of the antihistamine. A composition comprised of monodisperse conjugates can include, however, one or more nonconjugate substances such as solvents, reagents, excipients, and so forth.

"Bimodal," in reference to an oligomer composition, refers to an oligomer composition wherein substantially all oligomers in the composition have one of two definable and different numbers (as whole numbers) of monomers rather than a distribution, and whose distribution of molecular weights, when plotted as a number fraction versus molecular weight, appears as two separate identifiable peaks. Preferably, for a bimodal oligomer composition as described herein, each peak is generally symmetric about its mean, although the size of the two peaks may differ. Ideally, the polydispersity index of each peak in the bimodal distribution, Mw/Mn, is 1.01 or less, more preferably 1.001 or less, and even more preferably 1.0005 or less, and most preferably a MW/Mn value of 1.0000. By extension, a composition comprised of bimodal conjugates means that substantially all oligomers of all conjugates in the composition have one of two definable and different numbers (as whole numbers) of monomers rather than a large distribution and would possess a MW/Mn value of 1.01 or less, more preferably 1.001 or less and even more preferably 1.0005 or less, and most preferably a MW/Mn value of 1.0000 if the oligomer were not attached to the residue of the antihistamine. A composition comprised of bimodal conjugates can include, however, one or more nonconjugate substances such as solvents, reagents, excipients, and so forth.

An "antihistamine" is broadly used herein to refer to an organic, inorganic, or organometallic compound typically having a molecular weight of less than about 1000 Daltons (and typically less than 500 Daltons) and having some degree of activity as an antagonist at histamine- 1 receptors. An antihistamine is also referred to as an H1 receptor blocker or Hj receptor antagonist. The specific invention herein relates to diphenhydramine-related compounds.

A "biological membrane" is any membrane, typically made from specialized cells or tissues, that serves as a barrier to at least some foreign entities or otherwise undesirable materials. As used herein a "biological membrane" includes those membranes that are associated with physiological protective barriers including, for example: the blood-brain barrier (BBB); the blood-cerebrospinal fluid barrier; the blood-placental barrier; the blood-milk barrier; the blood-testes barrier; and mucosal barriers including the vaginal mucosa, urethral mucosa, anal mucosa, buccal mucosa, sublingual mucosa, rectal mucosa, and so forth. Unless the context clearly dictates otherwise, the term "biological membrane" does not include those membranes associated with the middle gastro-intestinal tract (e.g., stomach and small intestines).

A "biological membrane crossing rate," as used herein, provides a measure of a compound's ability to cross a biological membrane (such as the membrane associated with the blood-brain barrier). A variety of methods can be used to assess transport of a molecule across any given biological membrane. Methods to assess the biological membrane crossing rate associated with any given biological barrier (e.g., the blood-cerebrospinal fluid barrier, the blood-placental barrier, the blood-milk barrier, the intestinal barrier, and so forth), are known in the art, described herein and/or in the relevant literature, and/or can be determined by one of ordinary skill in the art.

A "reduced rate of metabolism" in reference to the present invention, refers to a measurable reduction in the rate of metabolism of a water-soluble oligomer-small molecule drug conjugate as compared to rate of metabolism of the small molecule drug not attached to the water-soluble oligomer (i.e., the small molecule drug itself) or a reference standard material. In the special case of "reduced first pass rate of metabolism," the same "reduced rate of metabolism" is required except that the small molecule drug (or reference standard material) and the corresponding conjugate are administered orally. Orally administered drugs are absorbed from the gastro-intestinal tract into the portal circulation and must pass through the liver prior to reaching the systemic circulation. Because the liver is the primary site of drug metabolism or biotransformation, a substantial amount of drug can be metabolized before it ever reaches the systemic circulation. The degree of first pass metabolism, and thus, any reduction thereof, can be measured by a number of different approaches. For instance, animal blood samples can be collected at timed intervals and the plasma or serum analyzed by liquid chromatography/mass spectrometry for metabolite levels. Other techniques for measuring a "reduced rate of metabolism" associated with the first pass metabolism and other metabolic processes are known in the art, described herein and/or in the relevant literature, and/or can be determined by one of ordinary skill in the art. Preferably, a conjugate of the invention can provide a reduced rate of metabolism reduction satisfying at least one of the following values: at least about 30%; at least about 40%; at least about 50%; at least about 60%; at least about 70%; at least about 80%; and at least about 90%. A compound (such as a small molecule drug or conjugate thereof) that is "orally bioavailable" is one that preferably possesses a bioavailability when administered orally of greater than 25%, and preferably greater than 70%, where a compound's bioavailability is the fraction of administered drug that reaches the systemic circulation in unmetabolized form.

"Alkyl" refers to a hydrocarbon chain, typically ranging from about 1 to 20 atoms in length. Such hydrocarbon chains are preferably but not necessarily saturated and may be branched or straight chain, although typically straight chain is preferred. Exemplary alkyl groups include methyl, ethyl, propyl, butyl, pentyl, 1-methylbutyl, 1-ethylpropyl, 3-methylpentyl, and the like. As used herein, "alkyl" includes cycloalkyl when three or more carbon atoms are referenced. An "alkenyl" group is an alkyl of 2 to 20 carbon atoms with at least one carbon-carbon double bond.

The terms "substituted alkyl" or "substituted C_{q-r} alkyl" where q and r are integers identifying the range of carbon atoms contained in the alkyl group, denotes the above alkyl groups that are substituted by one, two or three halo (e.g., F, Cl, Br, I), trifluoromethyl, hydroxy, C₁₋₇ alkyl (e.g., methyl, ethyl, n-propyl, isopropyl, butyl, t-butyl, and so forth), C₁₋₇ alkoxy, C₁₋₇ acyloxy, C₃₋₇ heterocyclic, amino, phenoxy, nitro, carboxy, carboxy, acyl, cyano. The substituted alkyl groups may be substituted once, twice or three times with the same or with different substituents.

"Lower alkyl" refers to an alkyl group containing from 1 to 6 carbon atoms, and may be straight chain or branched, as exemplified by methyl, ethyl, n-butyl, i-butyl, t-butyl. "Lower alkenyl" refers to a lower alkyl group of 2 to 6 carbon atoms having at least one carbon-carbon double bond.

"Non-interfering substituents" are those groups that, when present in a molecule, are typically non-reactive with other functional groups contained within the molecule.

"Alkoxy" refers to an -O-R group, wherein R is alkyl or substituted alkyl, preferably C₁-C₂₀ alkyl (e.g., methoxy, ethoxy, propyloxy, benzyl, etc.), preferably C₁-C₇.

"Pharmaceutically acceptable excipient" or "pharmaceutically acceptable carrier" refers to component that can be included in the compositions of the invention in order to provide for a composition that has an advantage (e.g., more suited for administration to a patient) over a composition lacking the component and that is recognized as not causing significant adverse toxicological effects to a patient.

The term "aryl" means an aromatic group having up to 14 carbon atoms. Aryl groups include phenyl, naphthyl, biphenyl, phenanthrenyl, naphthacenyl, and the like. "Substituted phenyl" and "substituted aryl" denote a phenyl group and aryl group, respectively, substituted with one, two, three, four or five (e.g. 1-2, 1-3 or 1-4 substituents) chosen from halo (F, Cl, Br, I), hydroxy, hydroxy, cyano, nitro, alkyl (e.g., C₁₋₆ alkyl), alkoxy (e.g., C₁₋₆ alkoxy), benzyloxy, carboxy, aryl, and so forth.

An "aromatic-containing moiety" is a collection of atoms containing at least aryl and optionally one or more atoms. Suitable aromatic-containing moieties are described herein.

For simplicity, chemical moieties are defined and referred to throughout primarily as univalent chemical moieties (e.g., alkyl, aryl, etc.). Nevertheless, such terms are also used to convey corresponding multivalent moieties under the appropriate structural circumstances clear to those skilled in the art. For example, while an "alkyl" moiety generally refers to a monovalent radical (e.g., CH₃-CH₂-), in certain circumstances a bivalent linking moiety can be "alkyl," in which case those skilled in the art will understand the alkyl to be a divalent radical (e.g., -CH₂-CH₂-), which is equivalent to the term "alkylene." (Similarly, in circumstances in which a divalent moiety is required and is stated as being "aryl," those skilled in the art will understand that the term "aryl" refers to the corresponding divalent moiety, arylene). All atoms are understood to have their normal number of valences for bond formation (i.e., 4 for carbon, 3 for N, 2 for O, and 2, 4, or 6 for S, depending on the oxidation state of the S).

"Pharmacologically effective amount," "physiologically effective amount," and "therapeutically effective amount" are used interchangeably herein to mean the amount of a water-soluble oligomer-small molecule drug conjugate present in a composition that is needed to provide a threshold level of active agent and/or conjugate in the bloodstream or in the target tissue. The precise amount will depend upon numerous factors, e.g., the particular active agent, the components and physical characteristics of the composition, intended patient population, patient considerations, and the like, and can readily be determined by one skilled in the art, based upon the information provided herein and available in the relevant literature.

A "difunctional" oligomer is an oligomer having two functional groups contained therein, typically at its termini. When the functional groups are the same, the oligomer is said to be homodifunctional. When the functional groups are different, the oligomer is said to be heterobifunctional.

A basic reactant or an acidic reactant described herein include neutral, charged, and any corresponding salt forms thereof.

The term "patient," refers to a living organism suffering from or prone to a condition that can be prevented or treated by administration of a conjugate as described herein, typically, but not necessarily, in the form of a water-soluble oligomer-small molecule drug conjugate, and includes both humans and animals.

"Optional" or "optionally" means that the subsequently described circumstance may but need not necessarily occur, so that the description includes instances where the circumstance occurs and instances where it does not.

Described herein in general is (among other things) a compound comprising a residue of an antihistamine covalently attached via a stable linkage to a water-soluble and non-peptidic oligomer.

For example, specific antihistamines include those selected from the group consisting of acrivastine, alimemazine, antazoline, astemizole, azatadine, azelastine, bamipine, bromazine, brompheniramine, bromodiphenhydramine, buclizine, carbinoxamine, cetirizine, chlorcyclizine, chloropyramine, chlorpheniramine, chlorphenoxamine, cinnarizine, clemastine, cyclizine, cyproheptadine, deptropine, desloratadine, dexbrompheniramine, dexchlorpheniramine, dimetindene, diphenylpyraline, doxylamine, dimenhydrinate, ebastine, histapyrrodine, hydroxyethylpromethazine, hydroxyzine, isothipendyl, ketotifen, loratadine, levocetirizine, mebhydrolin, meclizine, mepyramine, mequitazine, methapyrilene, methdilazine, mizolastine, niaprazine, oxomemazine, oxatomide, phenindamine, pheniramine, pimethixene, promethazine, pyribenzamine, pyrilamine, pyrrobutamine, rupatadine, talastine, terfenadine, thonzylamine, trimeprazine and tripelennamine. The present invention however relates to PEG conjugates of diphenhydramine.

It is believed that an advantage of the conjugates of the present invention is their ability to retain some degree of antihistaminic activity while not inducing clinically meaningful drowsiness. Although not wishing to be bound by theory, the extra size introduced by the oligomer - in contrast to the unconjugated "original" antihistamine - reduces the ability of the compound to cross the blood-brain barrier. In this way, the antihistaminic effects of the conjugate can act within the periphery while avoiding the central nervous system (and thereby avoid central nervous system-mediated side effects).

Use of oligomers (e.g., from a monodisperse or bimodal composition of oligomers, in contrast to relatively impure compositions) to form the conjugates of the invention can advantageously alter certain properties associated with the corresponding small molecule drug. For instance, a conjugate of the invention, when administered by any of a number of suitable administration routes, such as parenteral, oral, transdermal, buccal, pulmonary, or nasal, exhibits reduced penetration across the blood-brain barrier. It is preferred that the conjugate exhibit slowed, minimal or effectively no crossing of the blood-brain barrier, while still crossing the gastro-intestinal (GI) walls and into the systemic circulation if oral delivery is intended. Moreover, the conjugates of the invention maintain a degree of bioactivity as well as bioavailability in their conjugated form in comparison to the bioactivity and bioavailability of the compound free of all oligomers.

With respect to the blood-brain barrier ("BBB"), this barrier restricts the transport of drugs from the blood to the brain. This barrier consists of a continuous layer of unique endothelial cells joined by tight junctions. The cerebral capillaries, which comprise more than 95% of the total surface area of the BBB, represent the principal route for the entry of most solutes and drugs into the central nervous system.

For compounds whose degree of blood-brain barrier crossing ability is not readily known, such ability can be determined using a suitable animal model such as an *in situ* rat brain perfusion ("RBP") model as described herein. Briefly, the RBP technique involves cannulation of the carotid artery followed by perfusion with a compound solution under controlled conditions, followed by a wash out phase to remove compound remaining in the vascular space. (Such analyses can be conducted, for example, by contract research organizations such as Absorption Systems, Exton, PA). More specifically, in the RBP model, a cannula is placed in the left carotid artery and the side branches are tied off. A physiologic buffer containing the analyte (typically but not necessarily at a 5 micromolar concentration level) is perfused at a flow rate of about 10 mL/minute in a single pass perfusion experiment. After 30 seconds, the perfusion is stopped and the brain vascular contents are washed out with compound-free buffer for an additional 30 seconds. The brain tissue is then removed and analyzed for compound concentrations via liquid chromatograph with tandem mass spectrometry detection (LC/MS/MS). Alternatively, blood-brain barrier permeability can be estimated based upon a calculation of the compound's molecular polar surface area ("PSA"), which is defined as the sum of surface contributions of polar atoms (usually oxygens, nitrogens and attached hydrogens) in a molecule. The PSA has been shown to correlate with compound transport properties such as blood-brain barrier transport. Methods for determining a compound's PSA can be found, e.g., in, Ertl, P., et al., J. Med. Chem. 2000, 43, 3714-3717; and Kelder, J., et al, Pharm. Res. 1999, 16, 1514-1519.

With respect to the blood-brain barrier, the water-soluble, non-peptidic oligomer-small molecule drug conjugate exhibits a blood-brain barrier crossing rate that is reduced as compared to the crossing rate of the small molecule drug not attached to the water-soluble, non-peptidic oligomer. Preferred exemplary reductions in blood-brain barrier crossing rates for the compounds described herein include reductions of: at least about 30%; at least about 40%; at least about 50%; at least about 60%; at least about 70%; at least about 80%; or at least about 90%, when compared to the blood-brain barrier crossing rate of the small molecule drug not attached to the water-soluble oligomer. A preferred reduction in the blood-brain barrier crossing rate for a conjugate is at least about 20%.

As indicated above, the compounds described herein include a residue of an antihistamine, in particular diphenhydramine. Assays for determining whether a given compound (regardless of whether the compound is in conjugated form or not) can block H₁ antihistamine receptors are described infra.

In some instances, antihistamines described generally herein can be obtained from commercial sources. In addition, antihistamines can be obtained through chemical synthesis. Synthetic approaches for preparing antihistamines is described in the literature and in, for example, U.S. Patent Nos.: 2,421,714; 2,427,878; 4,525,358; 4,219,559; 2,567,245; 2,676,964, 3,061,517; 2,785,202; 2,951,082; 4,282,233; 2,709,169; 2,899,436; 2,406,594; and 2,502,151.

Each of these (and other) antihistamines can be covalently attached (either directly or through one or more atoms) to a water-soluble and non-peptidic oligomer.

Small molecule drugs generally have a molecular weight of less than 1000 Da. Exemplary molecular weights of small molecule drugs include molecular weights of: less than about 950; less than about 900; less than about 850; less than about 800; less than about 750; less than about 700; less than about 650; less than about 600; less than about 550; less than about 500; less than about 450; less than about 400; less than about 350; and less than about 300.

The small molecule drug, if chiral, may be in a racemic mixture, or an optically active form, for example, a single optically active enantiomer, or any combination or ratio of enantiomers (i.e., scalemic mixture), hi addition, the small molecule drug may possess one or more geometric isomers. With respect to geometric isomers, a composition can comprise a single geometric isomer or a mixture of two or more geometric isomers. A small molecule drug can be in its customary active form, or may possess some degree of modification. For example, a small molecule drug may have a targeting agent, tag, or transporter attached thereto, prior to or after covalent attachment of an oligomer. Alternatively, the small molecule drug may possess a lipophilic moiety attached thereto, such as a phospholipid (e.g., distearoylphosphatidylethanolamine or "DSPE," dipalmitoylphosphatidylethanolamine or "DPPE," and so forth) or a small fatty acid. In some instances, however, it is preferred that the small molecule drug moiety does not include attachment to a lipophilic moiety.

As described herein, antihistamine for coupling to a water-soluble and non-peptidic oligomer possesses a free hydroxyl, carboxyl, thio, amino group, or the like (i.e., "handle") suitable for covalent attachment to the oligomer. In addition, the antihistamine can be modified by introduction of a reactive group, preferably by conversion of one of its existing functional groups to a functional group suitable for formation of a stable covalent linkage between the oligomer and the drug. Both approaches are illustrated in the Experimental section.

The water-soluble and non-peptidic oligomer typically comprises one or more monomers serially attached to form a chain of monomers. The oligomer can be formed from a single monomer type (i.e., is homo-oligomeric) or two or three monomer types (i.e., is co-oligomeric). Preferably, each oligomer is a co-oligomer of two monomers or, more preferably, is a homo-oligomer. In accordance with the compound of the present invention, the number of monomers is 1 to 30 and the water-soluble polymer is polyethylene glycol.

However, as more generally described herein, each oligomer is composed of up to three different monomer types selected from the group consisting of: alkylene oxide, such as ethylene oxide or propylene oxide; olefinic alcohol, such as vinyl alcohol, 1-propenol or 2-propenol; vinyl pyrrolidone; hydroxyalkyl methacrylamide or hydroxyalkyl methacrylate, where alkyl is preferably methyl; [alpha]-hydroxy acid, such as lactic acid or glycolic acid; phosphazene, oxazoline, amino acids, carbohydrates such as monosaccharides, saccharide or mannitol; and N-acryloylmorpholine. Preferred monomer types include alkylene oxide, olefinic alcohol, hydroxyalkyl methacrylamide or methacrylate, N-acryloylmorpholine, and [alpha]-hydroxy acid. Preferably, each oligomer is, independently, a co-oligomer of two monomer types selected from this group, or, more preferably, is a homo-oligomer of one monomer type selected from this group.

The two monomer types in a co-oligomer may be of the same monomer type, for example, two alkylene oxides, such as ethylene oxide and propylene oxide. In the compound of the invention, the oligomer is a homo-oligomer of ethylene oxide. Usually, although not necessarily, the terminus (or termini) of the oligomer that is not covalently attached to a small molecule is capped to render it unreactive. Alternatively, the terminus may include a reactive group. When the terminus is a reactive group, the reactive group is either selected such that it is unreactive under the conditions of formation of the final oligomer or during covalent attachment of the oligomer to a small molecule drug, or it is protected as necessary. One common end-functional group is hydroxyl or -OH, particularly for oligoethylene oxides.

In accordance with the invention, the water-soluble, non-peptidic oligomer (e.g., "POLY" in various structures provided herein) can have any of a number of different geometries. For example, it can be linear, branched, or forked. Most typically, the water-soluble, non-peptidic oligomer is linear or is branched, for example, having one branch point. Although the invention herein is focused upon poly(ethylene oxide) as an illustrative oligomer, the discussion and structures presented herein can be readily extended to encompass any of the water-soluble, non-peptidic oligomers described above.

In accordance with the invention the molecular weight of the water-soluble, non-peptidic oligomer, excluding the linker portion, is generally relatively low. Exemplary values of the molecular weight of the water-soluble polymer include: below about 1500; below about 1450; below about 1400; below about 1350; below about 1300; below about 1250; below about 1200; below about 1150; below about 1100; below about 1050; below about 1000; below about 950; below about 900; below about 850; below about 800; below about 750; below about 700; below about 650; below about 600; below about 550; below about 500; below about 450; below about 400; below about 350; below about 300; below about 250; below about 200; and below about 100 Daltons.

Exemplary ranges of molecular weights of the water-soluble, non-peptidic oligomer (excluding the linker) include: from about 100 to about 1400 Daltons; from about 100 to about 1200 Daltons; from about 100 to about 800 Daltons; from about 100 to about 500 Daltons; from about 100 to about 400 Daltons; from about 200 to about 500 Daltons; from about 200 to about 400 Daltons; from about 75 to 1000 Daltons; and from about 75 to about 750 Daltons.

The number of monomers in the water-soluble, non-peptidic oligomer in accordance with the invention falls within one or more of the following ranges: between about 1 and about 30 (inclusive); between about 1 and about 25; between about 1 and about 20; between about 1 and about 15; between about 1 and about 12; between about 1 and about 10. hi certain instances, the number of monomers in series in the oligomer (and the corresponding conjugate) is one of 1, 2, 3, 4, 5, 6, 7, or 8. In additional embodiments, the oligomer (and the corresponding conjugate) contains 9, 10, 11, 12, 13, 14, 15, 16, 17, 18, 19, or 20 monomers. In yet further embodiments, the oligomer (and the corresponding conjugate) possesses 21, 22, 23, 24, 25, 26, 27, 28, 29 or 30 monomers in series. Thus, for example, when the water-soluble, non-peptidic oligomer includes CH3-(OCH2CH2),,-, "n" is an integer that can be 1, 2, 3, 4, 5, 6, 7, 8, 9, 10, 1 1, 12, 13, 14, 15, 16, 17, 18, 19, 20, 21, 22, 23, 24, 25, 26, 27, 28, 29 or 30, and can fall within one or more of the following ranges: between about 1 and about 25; between about 1 and about 20; between about 1 and about 15; between about 1 and about 12; between about 1 and about 10.

When the water-soluble, non-peptidic oligomer, as used in the invention, has 1, 2, 3, 4, 5, 6, 7, 8, 9, or 10 monomers, these values correspond to a methoxy end-capped oligo(ethylene oxide) having a molecular weights of about 75, 119, 163, 207, 251, 295, 339, 383,427, and 471 Daltons, respectively. When the oligomer has 11, 12, 13, 14, or 15 monomers, these values correspond to methoxy end-capped oligo(ethylene oxide) having molecular weights corresponding to about 515, 559, 603, 647, and 691 Daltons, respectively.

When the water-soluble and non-peptidic oligomer, as used in the invention, is attached to the antihistamine (in contrast to the step-wise addition of one or more monomers to effectively "grow" the oligomer onto the antihistamine), it is preferred that the composition containing an activated form of the water-soluble, non-peptidic oligomer be monodispersed. In those instances, however, where a bimodal composition is employed, the composition will possess a bimodal distribution centering around any two of the above numbers of monomers. Ideally, the polydispersity index of each peak in the bimodal distribution, Mw/Mn, is 1.01 or less, and even more preferably, is 1.001 or less, and even more preferably is 1.0005 or less. Most preferably, each peak possesses a MW/Mn value of 1.0000. For instance, a bimodal oligomer may have any one of the following exemplary combinations of monomer subunits: 1-2, 1-3, 1-4, 1-5, 1-6, 1-7, 1-8, 1-9, 1-10, and so forth; 2-3, 2-4, 2-5, 2-6, 2-7, 2-8, 2-9, 2-10, and so forth; 3-4, 3-5, 3-6, 3-7, 3-8, 3-9, 3-10, and so forth; 4-5, 4-6, 4-7, 4-8, 4-9, 4-10, and so forth; 4-5, 4-6, 4-7, 4-8, 4-9, 4-10, and so forth; 5-6, 5-7, 5-8, 5-9, 5-10, and so forth; 6-7, 6-8, 6-9, 6-10, and so forth; 7-8, 7-9, 7-10, and so forth; and 8-9, 8-10, and so forth.

In some instances, the composition containing an activated form of the water-soluble, non-peptidic oligomer, in accordance with the invention, will be trimodal or even tetramodal, possessing a range of monomers units as previously described. Oligomer compositions possessing a well- defined mixture of oligomers (i.e., being bimodal, trimodal, tetramodal, and so forth) can be prepared by mixing purified monodisperse oligomers to obtain a desired profile of oligomers (a mixture of two oligomers differing only in the number of monomers is bimodal; a mixture of three oligomers differing only in the number of monomers is trimodal; a mixture of four oligomers differing only in the number of monomers is tetramodal), or alternatively, can be obtained from column chromatography of a polydisperse oligomer by recovering the "center cut", to obtain a mixture of oligomers in a desired and defined molecular weight range.

It is preferred that the water-soluble, non-peptidic oligomer, in accordance with the invention, is obtained from a composition that is preferably unimolecular or monodisperse. That is, the oligomers in the composition possess the same discrete molecular weight value rather than a distribution of molecular weights. Some monodisperse oligomers can be purchased from commercial sources such as those available from Sigma- Aldrich, or alternatively, can be prepared directly from commercially available starting materials such as Sigma- Aldrich. Water-soluble, non- peptidic oligomers can be prepared as described in Chen Y., Baker, G. L., J. Org. Chem., 6870-6873 (1999), WO 02/098949, and U.S. Patent Application Publication 2005/0136031.

As described herein in general, when present, the spacer moiety (through which the water-soluble, non-peptidic polymer is attached to the antihistamine) may be a single bond, a single atom, such as an oxygen atom or a sulfur atom, two atoms, or a number of atoms. A spacer moiety is typically but is not necessarily linear in nature. The spacer moiety, "X" is preferably hydrolytically stable, and is preferably also enzymatically stable. Preferably, the spacer moiety "X" is one having a chain length of less than about 12 atoms, and preferably less than about 10 atoms, and even more preferably less than about 8 atoms and even more preferably less than about 5 atoms, whereby length is meant the number of atoms in a single chain, not counting substituents. For instance, a urea linkage such as this, Roiigomer-NH-(C=O)-NH- R'drug, is considered to have a chain length of 3 atoms (-NH-C(O)-NH-). In selected embodiments, the spacer moiety linkage does not comprise further spacer groups.

In some instances, the spacer moiety "X" comprises an ether, amide, urethane, amine, thioether, urea, or a carbon-carbon bond. Functional groups such as those discussed below, and illustrated in the examples, are typically used for forming the linkages. The spacer moiety may less preferably also comprise (or be adjacent to or flanked by) spacer groups, as described further below.

More specifically, a spacer moiety, X, may be any of the following: "-" (i.e., a covalent bond, that may be stable or degradable, between the residue of the small molecule antihistamine and the water-soluble, non-peptidic oligomer), -O-, -NH-, -S-, -C(O)-, C(O)-NH, NH-C(O)-NH, O-C(O)-NH, -C(S)-, -CH2-, -CH2-CH2-, -CH2-CH2-CH2-, -CH2-CH2-CH2-CH2-, -0-CH2-, -CH2-O-, -0-CH2-CH2-, -CH2-O-CH2-, -CH2-CH2-O-, -0-CH2-CH2-CH2-, -CH2-O-CH2-CH2-, -CH2-CH2-O-CH2-, -CH2-CH2-CH2-O-, -0-CH2-CH2-CH2-CH2-, -CH2-O-CH2-CH2-CH2-, -CH2-CH2-O-CH2-CH2-, -CH2-CH2-CH2-O-CH2-, -CH2-CH2-CH2-CH2-O-, -C(O)-NH-CH2-, -C(O)-NH-CH2-CH2-, -CH2-C(O)-NH-CH2-, -CH2-CH2-C(O)-NH-, -C(O)-NH-CH2-CH2-CH2-, -CH2-C(O)-NH-CH2-CH2-, -CH2-CH2-C(O)-NH-CH2-, -CH2-CH2-CH2-C(O)-NH-, -C(O)-NH-CH2-CH2-CH2-CH2-, -CH2-C(O)-NH-CH2-CH2-CH2-, -CH2-CH2-C(O)-NH-CH2-CH2-, -CH2-CH2-CH2-C(O)-NH-CH2-, -CH2-CH2-CH2-C(O)-NH-CH2-CH2-, -CH2-CH2-CH2-CH2-C(O)-NH -, -NH-C(O)-CH2-, -CH2-NH-C(O)-CH2-, -CH2-CH2-NH-C(O)-CH2-, -NH-C(O)-CH2-CH2-, -CH2-NH-C(O)-CH2-CH2, -CH2-CH2-NH-C(O)-CH2-CH2, -C(O)-NH-CH2-, -C(O)-NH-CH2-CH2-, -0-C(O)-NH-CH2-, -0-C(O)-NH-CH2-CH2-, -NH-CH2-, -NH-CH2-CH2-, -CH2-NH-CH2-, -CH2-CH2-NH-CH2-, -C(O)-CH2-, -C(O)-CH2-CH2-, -CH2-C(O)-CH2-, -CH2-CH2-C(O)-CH2-, -CH2-CH2-C(O)-CH2-CH2-, -CH2-CH2-C(O)-, -CH2-CH2-CH2-C(O)-NH-CH2-CH2-NH-, -CH2-CH2-CH2-C(O)-NH-CH2-CH2-NH-C(O)-, -CH2-CH2-CH2-C(O)-NH-CH2-CH2-NH-C(O)-CH2-, bivalent cycloalkyl group, -N(R<6>)-, R<6> is H or an organic radical selected from the group consisting of alkyl, substituted alkyl, alkenyl, substituted alkenyl, alkynyl, substituted alkynyl, aryl and substituted aryl.

For purposes of the present invention, however, a group of atoms is not considered a spacer moiety when it is immediately adjacent to an oligomer segment, and the group of atoms is the same as a monomer of the oligomer such that the group would represent a mere extension of the oligomer chain.

The linkage "X" between the water-soluble, non-peptidic oligomer and the small molecule is typically formed by reaction of a functional group on a terminus of the oligomer (or one or more monomers when it is desired to "grow" the oligomer onto the antihistamine) with a corresponding functional group within the antihistamine. General reactions are described briefly below. For example, an amino group on an oligomer may be reacted with a carboxylic acid or an activated carboxylic acid derivative on the small molecule, or vice versa, to produce an amide linkage. Alternatively, reaction of an amine on an oligomer with an activated carbonate (e.g. succinimidyl or benzotriazyl carbonate) on the drug, or vice versa, forms a carbamate linkage. Reaction of an amine on an oligomer with an isocyanate (R-N=C=O) on a drug, or vice versa, forms a urea linkage (R-NH-(C=O)-NH-R'). Further, reaction of an alcohol (alkoxide) group on an oligomer with an alkyl halide, or halide group within a drug, or vice versa, forms an ether linkage. In yet another coupling approach, a small molecule having an aldehyde function is coupled to an oligomer amino group by reductive amination, resulting in formation of a secondary amine linkage between the oligomer and the small molecule.

A particularly preferred water-soluble, non-peptidic oligomer is an oligomer bearing an aldehyde functional group. In this regard, the oligomer will have the following structure: CH3O-(CH2-CH2-O)n-(CH2)P-C(O)H, wherein (n) is one of 1, 2, 3, 4, 5, 6, 7, 8, 9 and 10 and (p) is one of 1, 2, 3, 4, 5, 6 and 7. Preferred (n) values include 3, 5 and 7 and preferred (p) values 2, 3 and 4. In addition, the carbon atom alpha to the -C(O)H moiety can optionally be substituted with alkyl.

Typically, the terminus of the water-soluble, non-peptidic oligomer not bearing a functional group is capped to render it unreactive. When the oligomer does include a further functional group at a terminus other than that intended for formation of a conjugate, that group is either selected such that it is unreactive under the conditions of formation of the linkage "X," or it is protected during the formation of the linkage "X."

As stated above, in general, the water-soluble, non-peptidic oligomer includes at least one functional group prior to conjugation. The functional group typically comprises an electrophilic or nucleophilic group for covalent attachment to a small molecule, depending upon the reactive group contained within or introduced into the small molecule. Examples of nucleophilic groups that may be present in either the oligomer or the small molecule include hydroxyl, amine, hydrazine (-NHNH2), hydrazide (-C(O)NHNH2), and thiol. Preferred nucleophiles include amine, hydrazine, hydrazide, and thiol, particularly amine. Most small molecule drugs for covalent attachment to an oligomer will possess a free hydroxyl, amino, thio, aldehyde, ketone, or carboxyl group.

Examples of electrophilic functional groups that may be present in either the oligomer or the small molecule include carboxylic acid, carboxylic ester, particularly imide esters, orthoester, carbonate, isocyanate, isothiocyanate, aldehyde, ketone, thione, alkenyl, acrylate, methacrylate, acrylamide, sulfone, maleimide, disulfide, iodo, epoxy, sulfonate, thiosulfonate, silane, alkoxysilane, and halosilane. More specific examples of these groups include succinimidyl ester or carbonate, imidazoyl ester or carbonate, benzotriazole ester or carbonate, vinyl sulfone, chloroethylsulfone, vinylpyridine, pyridyl disulfide, iodoacetamide, glyoxal, dione, mesylate, tosylate, and tresylate (2,2,2-trifluoroethanesulfonate).

Also included are sulfur analogs of several of these groups, such as thione, thione hydrate, thioketal, is 2-thiazolidine thione, etc., as well as hydrates or protected derivatives of any of the above moieties (e.g. aldehyde hydrate, hemiacetal, acetal, ketone hydrate, hemiketal, ketal, thioketal, thioacetal).

An "activated derivative" of a carboxylic acid refers to a carboxylic acid derivative which reacts readily with nucleophiles, generally much more readily than the underivatized carboxylic acid. Activated carboxylic acids include, for example, acid halides (such as acid chlorides), anhydrides, carbonates, and esters. Such esters include imide esters, of the general form -(CO)O-N[(CO)-]₂; for example, N-hydroxysuccinimidyl (NHS) esters or N-hydroxyphthalimidyl esters. Also preferred are imidazolyl esters and benzotriazole esters. Particularly preferred are activated propionic acid or butanoic acid esters, as described in co-owned U.S. Patent No. 5,672,662. These include groups of the form -(CH₂)₂₋₃C(=O)O-Q, where Q is preferably selected from N-succinimide, N-sulfosuccinimide, N-phthalimide, N-glutarimide, N-tetrahydrophthalimide, N-norbornene-2,3-dicarboximide, benzotriazole, 7-azabenzotriazole, and imidazole.

Other preferred electrophilic groups include succinimidyl carbonate, maleimide, benzotriazole carbonate, glycidyl ether, imidazoyl carbonate, p-nitrophenyl carbonate, acrylate, tresylate, aldehyde, and orthopyridyl disulfide.

These electrophilic groups are subject to reaction with nucleophiles, e.g. hydroxy, thio, or amino groups, to produce various bond types. Preferred for the present invention are reactions which favor formation of a hydrolytically stable linkage. For example, carboxylic acids and activated derivatives thereof, which include orthoesters, succinimidyl esters, imidazolyl esters, and benzotriazole esters, react with the above types of nucleophiles to form esters, thioesters, and amides, respectively, of which amides are the most hydrolytically stable. Carbonates, including succinimidyl, imidazolyl, and benzotriazole carbonates, react with amino groups to form carbamates. Isocyanates (R-N=C=O) react with hydroxyl or amino groups to form, respectively, carbamate (RNH-C(O)-OR') or urea (RNH-C(O)-NHR') linkages. Aldehydes, ketones, glyoxals, diones and their hydrates or alcohol adducts (i.e. aldehyde hydrate, hemiacetal, acetal, ketone hydrate, hemiketal, and ketal) are preferably reacted with amines, followed by reduction of the resulting imine, if desired, to provide an amine linkage (reductive amination).

Several of the electrophilic functional groups include electrophilic double bonds to which nucleophilic groups, such as thiols, can be added, to form, for example, thioether bonds. These groups include maleimides, vinyl sulfones, vinyl pyridine, acrylates, methacrylates, and acrylamides. Other groups comprise leaving groups that can be displaced by a nucleophile; these include chloroethyl sulfone, pyridyl disulfides (which include a cleavable S-S bond), iodoacetamide, mesylate, tosylate, thiosulfonate, and tresylate. Epoxides react by ring opening by a nucleophile, to form, for example, an ether or amine bond. Reactions involving complementary reactive groups such as those noted above on the oligomer and the small molecule are utilized to prepare the conjugates of the invention.

In some instances the antihistamine may not have a functional group suited for conjugation. In this instance, it is possible to modify the "original" antihistamine so that it does have the desired antihistamine. For example, if the antihistamine has an amide group, but an amine group is desired, it is possible to modify the amide group to an amine group by way of a Hofmann rearrangement, Curtius rearrangement (once the amide is converted to an azide) or Lossen rearrangement (once the amide is concerted to hydroxamide followed by treatment with tolyene-2-sulfonyl chloride/base).

It is possible to prepare a conjugate of small molecule antihistamine bearing a carboxyl group wherein the carboxyl group-bearing small molecule antihistamine is coupled to an amino-terminated oligomeric ethylene glycol, to provide a conjugate having an amide group covalently linking the small molecule antihistamine to the oligomer. This can be performed, for example, by combining the carboxyl group-bearing small molecule antihistamine with the amino-terminated oligomeric ethylene glycol in the presence of a coupling reagent, (such as dicyclohexylcarbodiimide or "DCC") in an anhydrous organic solvent.

Further, it is possible to prepare a conjugate of a small molecule antihistamine bearing a hydroxyl group wherein the hydroxyl group-bearing small molecule antihistamine is coupled to an oligomeric ethylene glycol halide to result in an ether (-O-) linked small molecule conjugate. This can be performed, for example, by using sodium hydride to deprotonate the hydroxyl group followed by reaction with a halide-terminated oligomeric ethylene glycol.

In another example, it is possible to prepare a conjugate of a small molecule antihistamine bearing a ketone group by first reducing the ketone group to form the corresponding hydroxyl group. Thereafter, the small molecule antihistamine now bearing a hydroxyl group can be coupled as described herein.

In still another instance, it is possible to prepare a conjugate of a small molecule antihistamine bearing an amine group. In one approach, the amine group-bearing small molecule antihistamine and an aldehyde-bearing oligomer are dissolved in a suitable buffer after which a suitable reducing agent (e.g., NaCNBH3) is added. Following reduction, the result is an amine linkage formed between the amine group of the amine group-containing small molecule antihistamine and the carbonyl carbon of the aldehyde-bearing oligomer.

In another approach for preparing a conjugate of a small molecule antihistamine bearing an amine group, a carboxylic acid-bearing oligomer and the amine group-bearing small molecule antihistamine are combined, typically in the presence of a coupling reagent (e.g., DCC). The result is an amide linkage formed between the amine group of the amine group-containing small molecule antihistamine and the carbonyl of the carboxylic acid-bearing oligomer.

The conjugates of the invention can exhibit a reduced blood-brain barrier crossing rate. Moreover, the conjugates maintain at least about 5%, 10%, 20%, 30%, 40%, 50%, 60%, 70%, or more of the bioactivity of the unmodified parent small molecule drug.

While it is believed that the full scope of the conjugates disclosed herein have been described, an optimally sized oligomer can be determined as follows.

First, an oligomer obtained from a monodisperse or bimodal water soluble oligomer is conjugated to the small molecule drug. Preferably, the drug is orally bioavailable, and on its own, exhibits a non-negligible blood-brain barrier crossing rate. Next, the ability of the conjugate to cross the blood-brain barrier is determined using an appropriate model and compared to that of the unmodified parent drug. If the results are favorable, that is to say, if, for example, the rate of crossing is significantly reduced, then the bioactivity of conjugate is further evaluated. Preferably, the compounds according to the invention maintain a significant degree of bioactivity relative to the parent drug, i.e., greater than about 30% of the bioactivity of the parent drug, or even more preferably, greater than about 50% of the bioactivity of the parent drug.

The above steps are repeated one or more times using oligomers of the same monomer type but having a different number of subunits and the results are compared.

For each conjugate whose ability to cross the blood-brain barrier is reduced in comparison to the non-conjugated small molecule drug, its oral bioavailability is then assessed. Based upon these results, that is to say, based upon the comparison of conjugates of oligomers of varying size to a given small molecule at a given position or location within the small molecule, it is possible to determine the size of the oligomer most effective in providing a conjugate having an optimal balance between reduction in biological membrane crossing, oral bioavailability, and bioactivity. The small size of the oligomers makes such screenings feasible, and allows one to effectively tailor the properties of the resulting conjugate. By making small, incremental changes in oligomer size, and utilizing an experimental design approach, one can effectively identify a conjugate having a favorable balance of reduction in biological membrane crossing rate, bioactivity, and oral bioavailability. In some instances, attachment of an oligomer as described herein is effective to actually increase oral bioavailability of the drug.

For example, one of ordinary skill in the art, using routine experimentation, can determine a best suited molecular size and linkage for improving oral bioavailability by first preparing a series of oligomers with different weights and functional groups and then obtaining the necessary clearance profiles by administering the conjugates to a patient and taking periodic blood and/or urine sampling. Once a series of clearance profiles have been obtained for each tested conjugate, a suitable conjugate can be identified.

Animal models (rodents and dogs) can also be used to study oral drug transport. In addition, non-*in vivo* methods include rodent everted gut excised tissue and Caco-2 cell monolayer tissue-culture models. In addition, the Experimental provides additional approaches to test oral drug transport. These models are useful in predicting oral drug bioavailability.

To determine whether the antihistamine of Formula I or the conjugate of an antihistamine and a water-soluble and non-peptidic polymer has binding activity to histamine-1 receptors, it is possible to test such a compound. In this regard, the Experimental *infra* includes a discussion for determining the binding activity of a compound to histamine-1 receptors.

With respect to antihistaminic activity, it is possible to determine whether the antihistamine of Formula I or the conjugate of an antihistamine and a water-soluble and non-peptidic polymer have activity as histamine-1 receptor antagonists. In one approach, the in vitro guinea pig ileum test is useful. Briefly, an isolated portion of the guinea pig ileum is secured under tension (500 mg) between an anchorage and a transducer in a 10 ml tissue bath and immersed in magnesium free Tyrode solution with constant aeration at a temperature of 30 °C. The output from the transducer is amplified. The amplified output is in turn fed to a flat bed recorder. Measured amounts of histamine are added to the tissue bath so that the histamine concentration increases step-wise until the force of the contraction reaches a maximum. The tissue bath is washed out and filled with fresh magnesium free Tyrode solution containing the compound of interest. The solution is left in contact with the tissue for 8 minutes and measured amounts of histamine are added again until a maximum contraction is recorded. The assay is repeated with increasing concentrations of test compound and the dose of histamine giving 50% of maximum contraction is noted. A dose ratio (DR) can be calculated by comparing the concentrations of histamine required to produce 50% maximum response in the absence and in the presence of the antagonist. A plot of Log DR-1 against Log D (the concentration of compound under test) is made and the point of intersection with the Log (DR-1) ordinate is taken as the measure of the activity (pA₂ value).

The present invention also includes pharmaceutical preparations comprising a conjugate as provided herein in combination with a pharmaceutical excipient. Generally, the conjugate itself will be in a solid form (e.g., a precipitate), which can be combined with a suitable pharmaceutical excipient that can be in either solid or liquid form.

Exemplary excipients include, without limitation, those selected from the group consisting of carbohydrates, inorganic salts, antimicrobial agents, antioxidants, surfactants, buffers, acids, bases, and combinations thereof.

A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like.

The excipient can also include an inorganic salt or buffer such as citric acid, sodium chloride, potassium chloride, sodium sulfate, potassium nitrate, sodium phosphate monobasic, sodium phosphate dibasic, and combinations thereof.

The preparation may also include an antimicrobial agent for preventing or deterring microbial growth. Examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the preparation as well. Antioxidants are used to prevent oxidation, thereby preventing the deterioration of the conjugate or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant may be present as an excipient. Exemplary surfactants include: polysorbates, such as "Tween 20" and "Tween 80," and pluronics such as F68 and F88 (both of which are available from BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; and chelating agents, such as EDTA, zinc and other such suitable cations.

Pharmaceutically acceptable acids or bases may be present as an excipient in the preparation. Examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of the conjugate in the composition will vary depending on a number of factors, but will optimally be a therapeutically effective dose when the composition is stored in a unit dose container. A therapeutically effective dose can be determined experimentally by repeated administration of increasing amounts of the conjugate in order to determine which amount produces a clinically desired endpoint.

The amount of any individual excipient in the composition will vary depending on the activity of the excipient and particular needs of the composition. Typically, the optimal amount of any individual excipient is determined through routine experimentation, i.e., by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99% by weight, preferably from about 5%-98% by weight, more preferably from about 15-95% by weight of the excipient, with concentrations less than 30% by weight most preferred.

These foregoing pharmaceutical excipients along with other excipients and general teachings regarding pharmaceutical compositions are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D.C., 2000.

The pharmaceutical compositions can take any number of forms and the invention is not limited in this regard. Exemplary preparations are most preferably in a form suitable for oral administration such as a tablet, caplet, capsule, gel cap, troche, dispersion, suspension, solution, elixir, syrup, lozenge, transdermal patch, spray, suppository, and powder.

Oral dosage forms are preferred for those conjugates that are orally active, and include tablets, caplets, capsules, gel caps, suspensions, solutions, elixirs, and syrups, and can also comprise a plurality of granules, beads, powders or pellets that are optionally encapsulated. Such dosage forms are prepared using conventional methods known to those in the field of pharmaceutical formulation and described in the pertinent texts.

Tablets and caplets, for example, can be manufactured using standard tablet processing procedures and equipment. Direct compression and granulation techniques are preferred when preparing tablets or caplets containing the conjugates described herein. In addition to the conjugate, the tablets and caplets will generally contain inactive, pharmaceutically acceptable carrier materials such as binders, lubricants, disintegrants, fillers, stabilizers, surfactants, coloring agents, and the like. Binders are used to impart cohesive qualities to a tablet, and thus ensure that the tablet remains intact. Suitable binder materials include, but are not limited to, starch (including corn starch and pregelatinized starch), gelatin, sugars (including sucrose, glucose, dextrose and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, microcrystalline cellulose, ethyl cellulose, hydroxyethyl cellulose, and the like), and Veegum. Lubricants are used to facilitate tablet manufacture, promoting powder flow and preventing particle capping (i.e., particle breakage) when pressure is relieved. Useful lubricants are magnesium stearate, calcium stearate, and stearic acid. Disintegrants are used to facilitate disintegration of the tablet, and are generally starches, clays, celluloses, algins, gums, or crosslinked polymers. Fillers include, for example, materials such as silicon dioxide, titanium dioxide, alumina, talc, kaolin, powdered cellulose, and microcrystalline cellulose, as well as soluble materials such as mannitol, urea, sucrose, lactose, dextrose, sodium chloride, and sorbitol. Stabilizers, as well known in the art, are used to inhibit or retard drug decomposition reactions that include, by way of example, oxidative reactions.

Capsules are also preferred oral dosage forms, in which case the conjugate-containing composition can be encapsulated in the form of a liquid or gel (e.g., in the case of a gel cap) or solid (including particulates such as granules, beads, powders or pellets). Suitable capsules include hard and soft capsules, and are generally made of gelatin, starch, or a cellulosic material. Two-piece hard gelatin capsules are preferably sealed, such as with gelatin bands or the like.

Included are parenteral formulations in the substantially dry form (typically as a lyophilizate or precipitate, which can be in the form of a powder or cake), as well as formulations prepared for injection, which are typically liquid and requires the step of reconstituting the dry form of parenteral formulation. Examples of suitable diluents for reconstituting solid compositions prior to injection include bacteriostatic water for injection, dextrose 5% in water, phosphate-buffered saline, Ringer's solution, saline, sterile water, deionized water, and combinations thereof.

In some cases, compositions intended for parenteral administration can take the form of nonaqueous solutions, suspensions, or emulsions, each typically being sterile. Examples of nonaqueous solvents or vehicles are propylene glycol, polyethylene glycol, vegetable oils, such as olive oil and corn oil, gelatin, and injectable organic esters such as ethyl oleate.

The parenteral formulations described herein can also contain adjuvants such as preserving, wetting, emulsifying, and dispersing agents. The formulations are rendered sterile by incorporation of a sterilizing agent, filtration through a bacteria-retaining filter, irradiation, or heat.

The conjugate can also be administered through the skin using conventional transdermal patch or other transdermal delivery system, wherein the conjugate is contained within a laminated structure that serves as a drug delivery device to be affixed to the skin. In such a structure, the conjugate is contained in a layer, or "reservoir," underlying an upper backing layer. The laminated structure can contain a single reservoir, or it can contain multiple reservoirs.

The conjugate can also be formulated into a suppository for rectal administration. With respect to suppositories, the conjugate is mixed with a suppository base material which is (e.g., an excipient that remains solid at room temperature but softens, melts or dissolves at body temperature) such as coca butter (theobroma oil), polyethylene glycols, glycerinated gelatin, fatty acids, and combinations thereof. Suppositories can be prepared by, for example, performing the following steps (not necessarily in the order presented): melting the suppository base material to form a melt; incorporating the conjugate (either before or after melting of the suppository base material); pouring the melt into a mold; cooling the melt (e.g., placing the melt-containing mold in a room temperature environment) to thereby form suppositories; and removing the suppositories from the mold.

The invention also provides the administration of a conjugate as provided herein to a patient suffering from a condition that is responsive to treatment with the conjugate. The treatment comprises administering, generally orally, a therapeutically effective amount of the conjugate (preferably provided as part of a pharmaceutical preparation). Other modes of administration are also contemplated, such as pulmonary, nasal, buccal, rectal, sublingual, transdermal, and parenteral. As used herein, the term "parenteral" includes subcutaneous, intravenous, intra-arterial, intraperitoneal, intracardiac, intrathecal, and intramuscular injection, as well as infusion injections.

In instances where parenteral administration is utilized, it may be necessary to employ somewhat bigger oligomers than those described previously, with molecular weights ranging from about 500 to 30K Daltons (e.g., having molecular weights of about 500, 1000, 2000, 2500, 3000, 5000, 7500, 10000, 15000, 20000, 25000, 30000 or even more).

The conjugate may be used to treat any condition that can be remedied or prevented by administration of the particular conjugate. Those of ordinary skill in the art appreciate which conditions a specific conjugate can effectively treat. The actual dose to be administered will vary depend upon the age, weight, and general condition of the subject as well as the severity of the condition being treated, the judgment of the health care professional, and conjugate being administered. Therapeutically effective amounts are known to those skilled in the art and/or are described in the pertinent reference texts and literature. Generally, a therapeutically effective amount will range from about 0.001 mg to 1000 mg, preferably in doses from 0.01 mg/day to 750 mg/day, and more preferably in doses from 0.10 mg/day to 500 mg/day.

The unit dosage of any given conjugate (again, preferably provided as part of a pharmaceutical preparation) can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, administration five times a day, four times a day, three times a day, twice daily, once daily, three times weekly, twice weekly, once weekly, twice monthly, once monthly, and any combination thereof. Once the clinical endpoint has been achieved, dosing of the composition is halted.

One advantage of administering the conjugates of the present invention is that a reduction in first pass metabolism may be achieved relative to the parent drug. Such a result is advantageous for many orally administered drugs that are substantially metabolized by passage through the gut. In this way, clearance of the conjugate can be modulated by selecting the oligomer molecular size, linkage, and position of covalent attachment providing the desired clearance properties. One of ordinary skill in the art can determine the ideal molecular size, linkage, and position of covalent attachment providing the desired clearance properties. One of ordinary skill in the art can determine the ideal molecular size of the oligomer based upon the teachings herein. Preferred reductions in first pass metabolism for a conjugate as compared to the corresponding nonconjugated small drug molecule include: at least about 10%, at least about 20%, at least about 30; at least about 40; at least about 50%; at least about 60%, at least about 70%, at least about 80% and at least about 90%.

Thus, the invention provides a method for reducing the metabolism of an active agent. The method comprises the steps of: providing monodisperse or bimodal conjugates, each conjugate comprised of a moiety derived from a small molecule drug covalently attached by a stable linkage to a water-soluble oligomer, wherein said conjugate exhibits a reduced rate of metabolism as compared to the rate of metabolism of the small molecule drug not attached to the water-soluble oligomer; and administering the conjugate to a patient. Typically, administration is carried out via one type of administration selected from the group consisting of oral administration, transdermal administration, buccal administration, transmucosal administration, vaginal administration, rectal administration, parenteral administration, and pulmonary administration.

Although useful in reducing many types of metabolism (including both Phase I and Phase II metabolism), the conjugates are particularly useful when the small molecule drug is metabolized by a hepatic enzyme (e.g., one or more of the cytochrome P450 isoforms) and/or by one or more intestinal enzymes.

### EXPERIMENTAL

All chemical reagents referred to in the appended examples are commercially available unless otherwise indicated. The preparation of PEG-mers is described in, for example, U.S. Patent Application Publication No. 2005/0136031.

All ¹H NMR (nuclear magnetic resonance) data was generated by a NMR spectrometer manufactured by Bruker (MHz ≥ 300).

### EXAMPLE 1

### Preparation of Diphenhydramine Conjugates

A schematic of one approach for synthesizing mPEGₙ-diphenydramine conjugates is provided in Scheme 1, below.

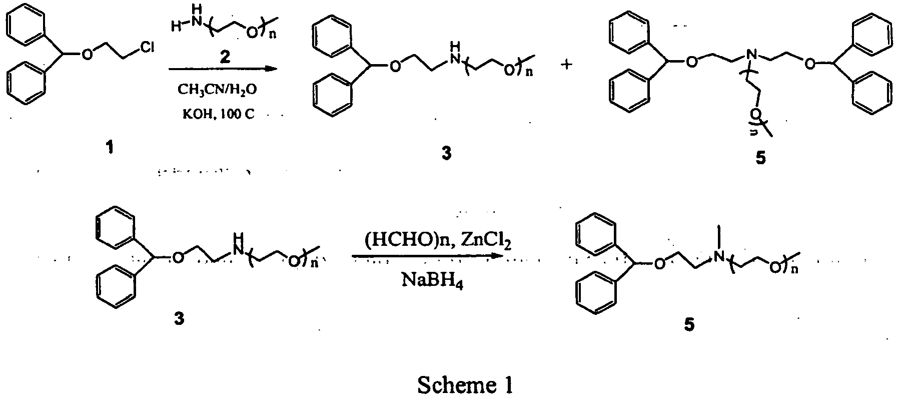

Synthesis of mPEGₙ-NH₂

In a round bottom flask 2 grams of mPEGₙ-OH were added to 1.7 mL triethylene amine. Dichloromethane (5mL) was then added. The solution was placed in an ice bath and allowed to stir for 30 minutes. Then, methanesulfonyl chloride (1.08 mL) was added to the reaction flask. The reaction was allowed to stir at room temperature overnight. Deionized water (15 mL) is added and the reaction mixture stirred for an additional 30 minutes. A separatory funnel was used to separate the layers. The organic layer was washed with 0.1N HCl (1 x 100mL) and water (1 x 100 mL), dried over sodium sulfate for two hours, filtered, and the solvent removed under reduced pressure. In a flask ammonium chloride (18 g) was added ammonium hydroxide (120 mL). The solid was allowed to dissolve at which point, mPEG-mesylate was added and allowed to stir for 48 hours at room temperature. Then, sodium chloride (18 g) was added to the flask and allowed to dissolve. The product was extracted with dichloromethane (3 x 100 mL). The organic layers were combined, dried over sodium sulfate, filtered, and the solvent removed under reduced pressure to give an oil.

Synthesis of mPEGₙ-NH-diphenhydramine

Benzhydryl 2-chloroethyl ether (2 mmol) and mPEGₙ-NH₂ (3 mmol) were dissolved in 10 ml of acetonitrile, and then sodium hydroxide (2 mmol) in water (1 mL) was added to the solution. The mixture was stirred at 100 °C for 16 hours. Dichloromethane (200 ml) was added to the reaction mixture, and the resulting solution was washed with water (200 mL x 3). The organic phase was dried and solvent was removed under reduced pressure. The crude product was purified by column chromatography (SiO₂: DCM/CH₃OH, 20:1) or alternatively using flash chromatography on silica gel using CAN/H₂O (40M C-18RP column, Biotage, Inc., Charlottesville, VA). The desired product of mPEGₙ-NH-diphenhydramine obtained in ∼70% yield and mPEGₙ-N-(diphenhydramine)₂ was also obtained in 15% yield.

mPEG₃-NH-diphenhydramine (3, n=3): ¹H NMR (300 MHz, CDCl₃): δ 7.34-7.25 *(m,* 10H), 5.39 (*s*, 1 H), 3.64-3.61 *(m,* 10H), 3.55 *(m,* 2H), 3.37 (s, 3H), 2.93 *(t,* 2H), 2.89 (*t*, 2H). ). LC-MS: 374.3 (MH⁺).

mPEG₄-NH-diphenhydramine (3, n=4): ¹H NMR (300 MHz, CDCl₃): δ 7.34-7.25 *(m,* 10H), 5.39 *(s,* 1H), 3.64-3.61 *(m,* 14H), 3.55 *(m,* 2H), 3.37 *(s,* 3H), 2.93 (*t*, 2H), 2.89 (*t*, 2H). ). LC-MS: 418.4 (MH⁺).

mPEG₅-NH-diphenhydramine (3, n=5): ¹H NMR (300 MHz, CDCl₃): δ 7.34-7.25 (*m*, 10H), 5.39 *(s,* 1H), 3.64-3.61 *(m,* 18H), 3.55 *(m,* 2H), 3.37 *(s,* 3H), 2.93 (*t*, 2H), 2.89 (*t*, 2H). ). LC-MS: 461.3 (MH⁺).

mPEG₆-NH-diphenhydramine (3, n=6): ¹H NMR (300 MHz, CDCl₃): δ 7.34-7.25 *(m,* 10H), 5.41 *(s,* 1H), 3.68-3.61 *(m,* 22H), 3.55 (*m*, 2H), 3.38 *(s,* 3H), 2.99 *(t,* 2H), 2.94 (*t*, 2H). LC-MS: Calc. 505.3; Found, 506.4 (MH⁺).

mPEG₇-NH-diphenhydramine (3, n=7): ¹H NMR (300 MHz, CDCl₃): δ 7.37-7.25 *(m,* 10H), 5.41 (s, 1H), 3.68-3.63 (*m*, 26H), 3.57 *(m,* 2H), 3.39 *(s,* 3H), 2.95 (*t*, 2H), 2.91 *(t,* 2H). LC-MS: Calc. 549.3; Found, 550.5 (MH⁺).

mPEGₙ-N-(diphenhydramine)₂ (4, n=6): ¹H NMR (300 MHz, CDCl₃): δ 7.36-7.23 *(m,* 20H), 5.35 *(s,* 2H), 3.65-3.60 *(m,* 16H), 3.53 *(m,* 10H), 3.39 (s, 3H), 2.90 (*t*, 4H), 2.81 (*t*, 2H). LC-MS: Calc. 715.4; Found, 716.4 (MH⁺).

Synthesis of mPEGₙ-N(CH₃)-diphenhydramine (5)

mPEGₙ-NH-Diphenhydramine (0.25 mmol), paraformaldehyde (0.5 mmol), and zinc chloride (0.5 mmol) was dissolved in 8 mL DCM. The mixture was stirred at room temperature for 1 hour before sodium borohydride (0.5 mmol) was added. The resulting reaction mixture was stirred overnight at room temperature. Dichloromethane (150 mL) was added into the reaction mixture, and the resulting solution was washed with water (150 mL × 4). The combined organic phases were dried and the solvent removed under reduced pressure. The crude product was purified by flash column chromatography on silica gel using DCM/MeOH (12M column, Biotage, Inc., Charlottesville, VA). The product was obtained in quantitative yield.

mPEG₃-N-diphenhydramine (5, n=3): ¹H-NMR (300 MHz, CDCl₃), δ 7.35-7.19 (*m*, 10 H, 2 Ph), 5.34 *(s,* 1 H, Ph₂CH), 3.62-3.50 *(m,* 12 H, 6 OCH₂), 3.35 (s, 3 H, OCH₃), 2.76 *(t, J=* 5.4-5.7 Hz, 2 H, NCH₂), 2.68 (*t*, *J* = 5.1-5.4 Hz, 2 H, NCH₂), 2.34 (s, 3 H, NCH₃); LC-MS: 388.3 (MH⁺).

mPEG₄-N-diphenhydramine **(5,** n=4): ¹H-NMR (300 MHz, CDCl₃), δ 7.35-7.19 *(m,* 10 H, 2 Ph), 5.34 (s, 1 H, Ph₂CH), 3.62-3.50 (m, 16 H, 8 OCH₂), 3.35 *(s,* 3 H, OCH₃), 2.76 *(t, J* = 5.4-5.7 Hz, 2 H, NCH₂), 2.68 *(t, J* = 5.1-5.4 Hz, 2 H, NCH₂), 2.34 *(s,* 3 H, NCH₃); LC-MS: 432.4 (MH⁺).

mPEG₅-N-diphenhydramine (5, n=5): ¹H NMR (300 MHz, CDCl₃): δ 7.38-7.25 *(m,* 10H), 5.39 *(s,* 1H), 3.67-3.61 *(m,* 20H), 3.40 *(s,* 3H), 2.80 (*t*, 2H), 2.72 (*t*, 2H), 2.38 (s, 3H). LC-MS: Calc. 475.3; Found. 476.4 (MH⁺)

mPEG₆-N-diphenhydramine (5, n=6): ¹H NMR (300 MHz, CDCl₃): δ 7.38-7.25 (*m*, 10H), 5.38 *(s,* 1H), 3.67-3.60 *(m,* 24H), 3.39 (s, 3H), 2.76 (*t*, 2H), 2.68 (*t*, 2H), 2.35 (s, 3H). LC-MS: Calc. 519.3; Found, 520.4 (MH⁺).

mPEG₇-N-diphenhydramine (5, n=7): ¹H NMR (300 MHz, CDCl₃): δ 7.38-7.25 (*m*, 10H), 5.38 *(s,* 1H), 3.67-3.60 (*m*, 28H), 3.39 *(s,* 3H), 2.76 (*t*, 2H), 2.68 (*t*, 2H), 2.35 (*s*, 3H). LC-MS: Calc. 563.3; Found, 564.5 (MH⁺).

Synthesis of diphenhydramine conjugates intended for scale up.

Scale-up the above-mentioned PEG-diphenhydramine conjugates to support in vivo studies were seen as sub-optimal inasmuch as the reaction temperature was needed to be raised to 120°C, chromatography after certain steps resulted in a lower overall yield, and there was difficulty in achieving and sustaining anhydrous conditions.

In an alternative approach for synthesizing conjugates, commercially available benzhydrol and 2-bromoethanol were used, in accordance with the synthetic scheme (scheme 2) shown below.

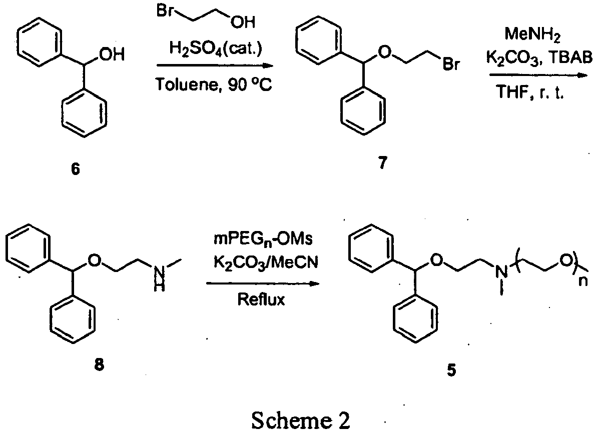

In this approach, benzhydrol (6) was reacted with 2-bromoethanol in toluene at 90 °C in the presence of concentrated sulfuric acid to afford 2-bromoethylbenzhydryl ether (7). 2-Bromoethyl benzhydryl ether (7) was converted to (2-benzhydryloxy ethyl) methylamine **(8)** by reaction with methylamine in THF at room temperature ("r.t.") in the presence of potassium carbonate as the base and tetrabutylammonium bromide ("TBAB") as a phase transfer catalyst. (2-Benzhydryloxy ethyl) methylamine **(8)** was reacted with mPEGₙ-OMs (wherein "Ms" is mesylate) in the presence of potassium carbonate as the base giving the final product mPEGₙ-N-diphenhydramine **(5).**

Synthesis of Benzhydryl 2-bromoethyl ether **(7)**

Concentrated sulfuric acid (1.7 mL) was added to a stirred solution of 2-bromoethanol (1.042 g, 76.34 mmol) in toluene (70 mL) at room temperature. The solution was warmed to 60 °C. Benzhydrol (9.623 g, 51.71 mmol) in warm toluene (65 mL) was added dropwise over 20 minutes. During the addition, the solution became cloudy. The reaction mixture was maintained at 90 °C for 6.5 hours, cooled to room temperature, and then diluted with toluene (50 mL). The mixture was poured into ice-water bath. The organic phase was separated and washed with 5% aqueous sodium bicarbonate (100 mL), brine (2 x 200 mL), dried over Na₂SO₄, and concentrated under reduced pressure. The resulting residue was purified by flash column chromatography on silica gel using 3-8 % EtOAc/Hexane (40M column, Biotage, Inc., Charlottesville, VA) to afford 11.64 g of product in 77% yield. Note: The product could be purified by distillation under reduced pressure (b. p. 125 °-165 °C/2.5 mmHg). ¹H-NMR (300 MHz, CDCl₃), δ 7.35-7.19 (m, 10 H, 2 Ph), 5.38 (*s*, 1 H, Ph₂CH), 3.77 (*t, J=* 6.0-6.3 Hz, 2 H, OCH₂), 3.51 (*t*, *J=* 6.0-6.3 Hz, 2 H, CH₂Br).

Synthesis of 2-benzhydryloxy ethyl) methylamine **(8)**

A mixture of benzhydryl 2-bromoethyl ether 7 (3.35 g, 11.50 mmol), potassium carbonate (10.13 g, 72.57 mmol), tetrabutylammonium bromide (652 mg, 2.0 mmol) in methylamine (2.0 M THF solution, 63 mL, 126 mmol) was stirred at room temperature for 27.5 hours. Water was added to quench the reaction, concentrated to remove the organic solvent and excess of methylamine. The remaining aqueous solution was extracted with dichloromethane (4 x 30 mL). The combined organic solution was washed with brine (2 x 40 mL), dried over Na₂SO₄, and concentrated under reduced pressure. The resulting residue was purified by flash column chromatography on silica gel using 3-8% MeOH/DCM (40M column, 20 CV, Biotage, Inc., Charlottesville, VA) to afford the product (1.93 g) in 69% yield. ¹H-NMR (300 MHz, CDCl₃), δ 7.35-7.19 *(m,* 10 H, 2 Ph), 5.36 *(s,* 1 H, Ph₂CH), 3.61 (*t, J* = 5.1 Hz, 2 H, OCH₂), 2.84 *(t, J* = 5.1 Hz, 2 H, NCH₂), 2.43 (s, 3 H, NCH₃); LC-MS: 242.1 (MH⁺).

Synthesis of mPEG₃-N-diphenhydramine **(5,** n=3)

A mixture of (2-benzhydryloxy ethyl) methylamine **8** (280 mg, 1.16 mmol) and mPEG₃-OMs (252 mg, 1.04 mmol, wherein "Ms" represents mesylate) in acetonitrile (20 mL) in the presence of potassium carbonate (441 mg, 3.16 mmol) was stirred at room temperature for 60 minutes, and then heated to reflux for 23 hours. The mixture was cooled to room temperature, filtered and the solid washed with DCM. The collected organic solution was concentrated under reduced pressure and the residue was purified by flash column chromatography on silica gel using 0-10% MeOH/DCM (25M column, 20 CV, Biotage, Inc., Charlottesville, VA) to afford the product (300 mg) in 74% yield. ¹H-NMR (300 MHz, CDCl₃), δ 7.35-7.19 *(m,* 10 H, 2 Ph), 5.34 (s, 1 H, Ph₂CH), 3.62-3.50 *(m,* 12 H, 6 OCH₂), 3.35 (s, 3 H, OCH₃), 2.76 *(t, J=* 5.4-5.7 Hz, 2 H, NCH₂), 2.68 (*t*, *J* = 5.1-5.4 Hz, 2 H, NCH₂), 2.34 (s, 3 H, NCH₃); LC-MS: 388.3 (MH⁺).

mPEG₆-N-diphenhydramine (5, n=6)

A mixture of (2-benzhydryloxy ethyl) methylamine **8** (228 mg, 0.95 mmol) and mPEG₆-OMs (410 mg, 1.10 mmol) in acetonitrile (15 mL) in the presence of potassium carbonate (399 mg, 2.86 mmol) was stirred at room temperature for 90 minutes, and then heated to reflux for 22.5 hours. Additional quantities of mPEG₆-OMs (100mg, 0.27 mmol) were added. The mixture was heated to reflux for another 23 hours. The mixture was cooled to room temperature, filtered and the solid washed with acetonitrile and DCM. The collected organic solution was concentrated under reduced pressure and the residue was purified by flash column chromatography on silica gel using 3-10% MeOH/DCM (25M column, 20 CV, Biotage, Inc., Charlottesville, VA) to afford the product (308 mg) in 63% yield. ¹H-NMR (300 MHz, CDCl₃), δ 7.35-7.18 (m, 10 H, 2 Ph), 5.34 (*s,* 1H, Ph₂CH), 3.70-3.50 (m, 24 H, 12 OCH₂), 3.35 (*s*, 3 H, OCH₃), 2.76 (*t, J =* 5.7-6.0 Hz, 2 H, NCH₂), 2.68 *(t, J =* 5.7-6.0 Hz, 2 H, NCH₂), 2.34 (*s*, 3 H, NCH₃); LC-MS: 520.4 (MH⁺).

An additional synthetic approach was used, a schematic of which is provided below "as Scheme 3."

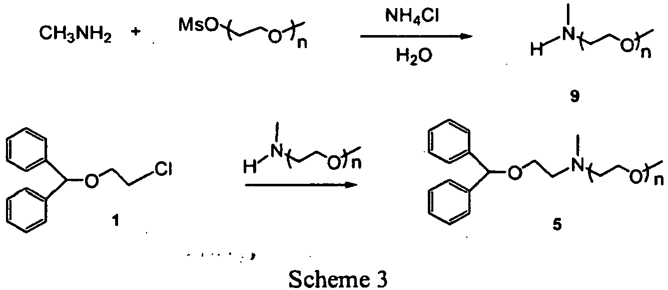

In this approach, preparation of mPEGₙ₋N-Me **(9)** was performed by reacting the activated mPEGₙ-OMs with methylamine using ammonium chloride as a catalyst. The mPEGₙ-N-Me was subsequently reacted with the commercially available benzhydryl 2-chloroethyl ether **(1)** to give the desired mPEGn-N-diphenhydramine conjugates in only 2 steps with yields comparable to those obtained in the synthetic pathway outlined in Scheme 1.

Synthesis of mPEGₙ-OMs **(9)**

In a round bottom flask mPEGₙ-OH (4.00 g) was added to triethylamine (3.39 mL). Then, dichloromethane (10 mL) was added and the solution was placed in an ice bath and allowed to stir for 30 minutes. Then, methanesulfonyl chloride (2.16 mL) was added to the reaction flask. The reaction was allowed to stir overnight and then deionized water (15 mL) was added to the reaction mixture. The solution stirred for an additional 30 minutes. Then, dichloromethane (40 mL) was added. A separatory funnel was used to separate the layers and 0.1N HCl (100 mL) was added. The organic layer was collected and washed with deionized water (3 x 100 mL), dried over Na₂SO₄, filtered, and the solvent removed under reduced pressure to give the desired mPEGₙ-OMs as an oil.

Synthesis of mPEGₙN-CH₃ **(9)**

In a round bottom flask ammonium chloride (30 g) was dissolved in ammonium hydroxide (200 mL). Then, the above-prepared mPEGₙ-OMs (3.8 g) was added and the reaction mixture was allowed to stir for 48 hrs. The product was extracted with dichloromethane (3 x 100 mL). The combined organic extracts were dried over Na₂SO₄, filtered, and the solvent removed under reduced pressure. The desired product was obtained as an oil and was not placed under the vacuum due to the observance of decomposition after 2 hours. The product was comfirmed by ¹H NMR.

Preparation of mPEGₙ-N-diphenhydramine **(5)**

Benzhydryl 2-chloroethyl ether **(1)** was dissolved in acetonitrile (10 mL) and added to mPEGₙ-N-CH₃ **(9,** 370 g). Then a solution of sodium hydroxide/water (160 mg) was added with stirring. The reaction mixture was stirred overnight at 120 °C and then dichloromethane (400 mL) was added to the solution. The organic layer washed with (3 x 300 mL), NaCl/H₂O (1 x 300 mL), dried over Na₂SO₄ for 2 hours, filtered, and the solvent removed under reduced pressure. The resulting product was purified by flash column chromatography on silica gel using MeOH/DCM (25M column, Biotage, Inc., Charlottesville, VA) to afford the desired product.

mPEG₃-N-diphenhydramine **(5,** n=3): ¹H-NMR (300 MHz, CDCl₃), δ 7.35-7.19 *(m,* 10 H, 2 Ph), 5.34 *(s,* 1 H, Ph₂CH), 3.62-3.50 (*m*, 12 H, 6 OCH₂), 3.35 (s, 3 H, OCH₃), 2.76 (*t, J=* 5.4-5.7 Hz, 2 H, NCH₂), 2.68 (*t, J=* 5.1-5.4 Hz, 2 H, NCH₂), 2.34 *(s,* 3 H, NCH₃); LC-MS: 388.3 (MH⁺).

mPEG₅-N-diphenhydramine **(5,** n=5): ¹H NMR (300 MHz, CDCl₃): δ 7.38-7.25 (*m*, 10H), 5.39 *(s,* 1H), 3.67-3.61 (*m*, 20H), 3.40 *(s,* 3H), 2.80 (*t*, 2H), 2.72 (*t*, 2H), 2.38 (*s*, 3H). LC-MS: Calc. 475.3; Found. 476.4 (MH⁺).

mPEG6-N-diphenhydramine (5, n=6): is NMR (300 MHz, CDCl₃): [delta] 7.38-7.25 (m, 10H), 5.38 (s, 1H), 3.67-3.60 (m, 24H), 3.39 (s, 3H), 2.76 (t, 2H), 2.68 (t, 2H), 2.35 (s, 3H). LC-MS: Calc. 519.3; Found, 520.4 (MH<+>).

mPEG7-N-diphenhydramine (5, n=7): <1>H NMR (300 MHz, CDCl3): [delta] 7.38-7.25 (m, 10H), 5.38 (s, 1H), 3.67-3.60 (m, 28H), 3.39 (s, 3H), 2.76 (t, 2H), 2.68 (t, 2H), 2.35 (s, 3H). LC-MS: Calc. 563.3; Found, 564.5 (MH<+>).
Inclusion herein of synthesis and testing of hydroxyzine and cetrizine is for comparison and understanding of the invention.

### Reference EXAMPLE 2

### Preparation of Hydroxyzine Conjugates

Schematic representation of preparation of mPEGn-Br (n = 1, 3, 5, 6, 7, 8)

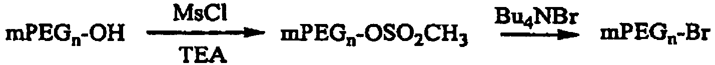

Synthetic procedure for preparing hydroxyzine conjugates using mPEG5-Br:

mPEG(n=5)-mesylate: Triethylamine (5.7 mL, 40 mmol) was added into mPEG(n=5)-OH (5.0 g, 20 mmol) in dichloromethane (20mL) with stirring. The solution was cooled in an ice bath under N2, and 2.5 mL of methanesulfonyl chloride (32 mmol) was added dropwise in 30 minutes. The solution was then stirred overnight at room temperature. 40 mL of water was added into reaction mixture and the solution was extracted with CH2Cl2 (3*150 mL), the organic phase was washed with 0.1 N HCl (3*80 mL) and water (2*80 mL). After drying with Na2SO4 and removing the solvent under reduced pressure, the desired mPEG(n=5)-mesylate was obtained as a light brown liquid in quantitative yield. <1>H NMR (300 Hz, CDCl₃): [delta] 4.41 (m, 2H), 3.80 (m, 2H), 3.71 (m, 14H), 3.58(m, 2H), 3.41 (s, 3H), 3.11 (s, 3H).

mPEG(n=5)-Br: mPEG(n=5)-Mesylate (6.51 g,19.8 mmol) and Bu4NBr (12.80 g, 39.7 mmol) were dissolved in CH3CN (50 mL), and the resulting solution was stirred under N2 at 50[deg.]C for 15 hours. After cooling to room temperature, CH3CN was removed under reduced pressure to give a red liquid, which was dissolved in 150 mL water and extracted with EtOAc (2*200 mL). The organic phases were combined, washed with water, and dried over Na2SO4. After the removal of solvent, a red liquid was obtained (4.83 g, 77.4%). <1>H NMR (300 Hz, CDCl3): [delta] 3.82 (t, 2H), 3.67 (m, 14H), 3.51 (m, 2H), 3.40 (s, 3H).

Following the above procedure, mPEGₙ-Br with values other than five were prepared.

Schematic of the general synthesis of mPEGₙ-hydroxyzine conjugates (n = 1, 3, 5, 6, 7, 8)

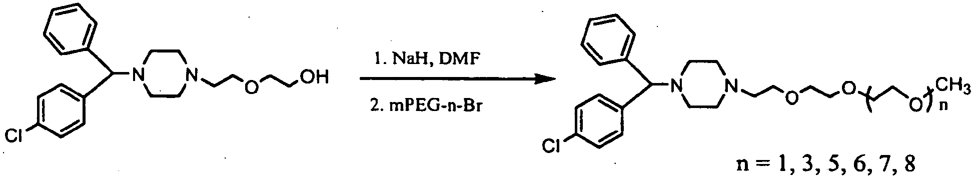

Hydroxyzine dihydrochloride (1.0 mmol) was dissolved in 6 mL DMF. To the solution, NaH (5.0 mmol) was added with stirring. After 20 minutes, mPEGₙ-Br (3.0 mmol) was added to the solution, which was then stirred overnight at room temperature. Dichloromethane (150 mL) was added and the precipitated solid was collected by filtration. The organic filtrate was washed with water (100 mL x 2) and then dried. The crude product was purified by column chromatography (SiO₂: DCM/Methanol 20:1). The products were obtained as light-yellow oil in high yield and purity (yield: ∼90%, purity: > 99%).

mPEG₁-Hydroxyzine: ¹H NMR (300 Hz, CDCl₃): δ 7.40 (m, 4H), 7.28 (m, 5H), 4.23 (s, 1H), 3.65 (m, 8H), 3.57 (m, 2H), 3.40 (s, 3H), 2.64 (t, 2H), 2.61(b, 4H), 2.57 (b, 4H).

mPEG₃-Hydroxyzine: ¹H NMR (300 Hz, CDCl3): δ 7.38 (m, 4H), 7.28 (m, 5H), 4.20 (s, 1H), 3.64 (m, 18H), 3.42 (s, 3H), 2.64 (t, 2H), 2.61(b, 4H), 2.57 (b, 4H). LC-MS: Calc. 520.3, Found. 521.3 (M + H⁺)

mPEG₅-Hydroxyzine: ¹H NMR (300 Hz, CDCl3): δ 7.36 (m, 4H), 7.24 (m, 5H), 4.21 (s, 1H), 3.63 (m, 26H), 3.39 (s, 3H), 2.61 (t, 2H), 2.55(b, 4H), 2.43 (b, 4H). LC-MS: Calc. 608.3, Found. 609.3 (M + H⁺)

mPEG₆-Hydroxyzine: ¹H NMR (300 Hz, CDCl₃): δ 7.36 (m, 4H), 7.24 (m, 5H), 4.19 (s, 1H), 3.63 (m, 30H), 3.39 (s, 3H), 2.61 (t, 2H), 2.55(b, 4H), 2.43 (b, 4H).

mPEG₇-Hydroxyzine: ¹H NMR (300 Hz, CDCl3): δ 7.38 (m, 4H), 7.28 (m, 5H), 4.20 (s, 1H), 3.64 (m, 34H), 3.42 (s, 3H), 2.64 (t, 2H), 2.61(b, 4H), 2.57 (b, 4H). LC-MS: Calc. 696.4, Found. 697.4 (M + H⁺)

mPEG₈-Hydroxyzine: ¹H NMR (300 Hz, CDCl3): δ 7.36 (m, 4H), 7.24 (m, 5H), 4.21 (s, 1H), 3.63 (m, 38H), 3.39 (s, 3H), 2.61 (t, 2H), 2.55(b, 4H), 2.43 (b, 4H).

### Reference Example 3

### Preparation of Cetirizine Conjugates

Schematic representation of preparation of mPEGₙ-NH₂ is provided immediately below.

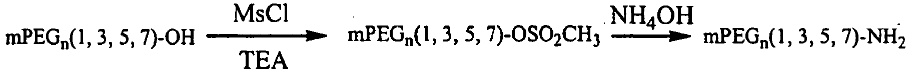

Schematic representation of preparation of cetirizine conjugate is provided immediately below.

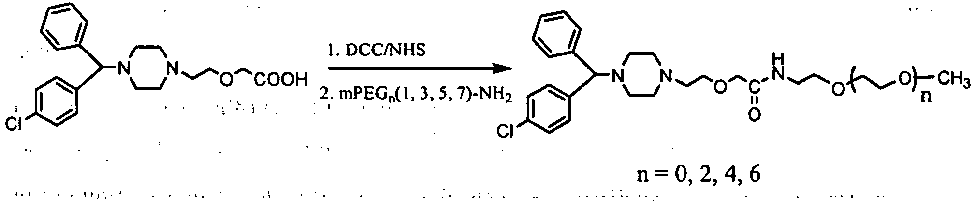

Synthetic procedure for preparing mPEGₙ-Cetirizine

Cetirizine dihydrochloride (1.0 mmol) was dissolved in 8 mL DMF. DCC (1.1 mmol) and N-hydroxysuccinimide (1.1 mmol) were added to the solution. The mixture was stirred overnight at room temperature and then mPEGₙ-NH₂ (5 mmol) was added. The reaction was continued for another 5 hours. The precipitated solid was removed by filtration and the solvent removed under reduced pressure. The resulting residue was purified by column chromatography giving the desired products in ∼80% yield with a purity of >99% (HPLC).

mPEG₁-Cetirizine: δ 7.42 (m, 1H), 7.40 (m, 4H), 7.28 (m, 5H), 4.25 (s, 1 H), 3.99 (s, 2H), 3.65 (t, 2H), 3.48 (m, 4H), 3.34 (s, 3H), 2.63 (t, 2H), 2.58(b, 4H), 2.46 (b, 4H); Analytical HPLC: t_{R} = 7.15 min.

mPEG₃-Cetirizine: δ 7.45 (m, 1H), 7.35 (m, 4H), 7.25 (m, 5H), 4.22 (s, 1H), 3.96 (s, 2H), 3.59 (m, 10H), 3.53 (m, 2H), 3.46 (m, 2H), 3.37 (s, 3H), 2.60 (t, 2H), 2.58 (b, 4H), 2.43 (b, 4H); Analytical HPLC: t_{R} = 7.22 min.

mPEG₅-Cetirizine: δ 7.42 (m, 1H), 7.36 (m, 4H), 7.24 (m, 5H), 4.23 (s, 1H), 3.96 (s, 2H), 3.60 (m, 20H), 3.46 (m, 2H), 3.38 (s, 3H), 2.60 (t, 2H), 2.55(b, 4H), 2.43 (b, 4H); Analytical HPLC: t_{R} = 7.28 min.

mPEG₇-Cetirizine: δ 7.45 (m, 1H), 7.35 (m, 4H), 7.25 (m, 5H), 4.22 (s, 1H), 3.96 (s, 2H), 3.59 (m, 28H), 3.46 (m, 2H), 3.37 (s, 3H), 2.60 (t, 2H), 2.58 (b, 4H), 2.43 (b, 4H).

### Example 4

### Blood-Brain Barrier ("BBB") Assay

The BBB model

The in situ brain perfusion technique utilized intact rat brain and allowed for the determination of drug permeation across the BBB under normal physiological conditions. The model also allowed for the study of transport mechanisms such as passive diffusion versus carrier mediated transport. Perfusion was performed using the single time-point method. The perfusion fluid (perfusate) containing the test compound(s) was infused into rats via the left external carotid artery at a constant rate by an infusion pump (20mL/min). The perfusion flow rate was set to completely take over fluid flow to the brain at normal physiologic pressure (80-120 mm Hg). The duration of the perfusion was 30 seconds. Immediately following the perfusion, the brain vasculature was perfused for an additional 30 seconds with drug-free perfusate to remove residual drug. The pump was turned off and the brain was then immediately removed from the skull. Left-brain samples from each rat were first weighed and then homogenized using a Polytron homogenizer. Four (4) mL of 20% methanol was added to each rat brain for homogenization. After homogenization, the total volume of homogenate was measured and recorded.

A measured amount of the homogenate diluted with organic solvent and centrifuged. The supernatant was removed, evaporated in a stream of nitrogen and reconstituted and analyzed by LC/MS/MS. Quantification of drug concentrations in brain homogenate was done against calibration curves generated by spiking the drugs into blank (i.e. drug-free) brain homogenate. Analysis of the drug concentrations in brain homogenates was done in triplicate.

Each perfusion solution contained atenolol (target concentration, 50 µM), antipyrine (target concentration, 5 µM) and a test compound (from list above) at a target concentration of 20 µM.

**Table I**

| **Histamine (H₁) Binding Activity** | | | |
|---|---|---|---|
| **Drug** | **Molecular Weight** | **Solubility (µM)** | **IC₅₀** |
| Hydroxyzine | 374.91 | soluble as HCl salt | 48.8 nM |
| PEG₁-Hydroxyzine | 433.0 | soluble | 70.3 nM |
| PEG₃-Hydroxyzine | 521.0 | soluble | 105.0 nM |
| PEG₅-Hydroxyzine | 609.0 | soluble | 76.7 nM |
| PEG₇-Hydroxyzine | | | Not tested |
| Cetirizine | 388.89 | soluble as HCl salt | 77.1 nM |
| PEG₁-Cetirizine | 446.0 | soluble | 61.0 nM |
| PEG₃-Cetirizine | 534.0 | soluble | 86.4 nM |
| PEG₅-Cetirizine | 622.0 | soluble | 128.0 nM |
| PEG₇-Cetirizine | | | Not tested |
| Diphenhydramine | - | - | 4.32 10⁻⁸ M |
| mPEG₅-N-Diphenhydramine | - | - | 60.0 10⁻⁸ M |
| mPEG₆-N-Diphenhydramine | - | - | 59.1 10⁻⁸ M |
| mPEG₇-N-Diphenhydramine | - | - | 173 10⁻⁸ M |
| mPEG₆-NH-Diphenhydramine | - | - | 412 10⁻⁸ M |
| mPEG₇-NH-Diphenhydramine | - | - | 761 10⁻⁸ M |
| mPEG₆-N-(Diphenhydramine)₂ | - | - | 650 10⁻⁸ M |

**Table II**

| **Brain Uptake Rate** | | |
|---|---|---|
| **Drug** | **Normalized Brain Uptake Rate in pmole/gm brain/sec (Mean ± SD)** | **N (rats)** |
| Atenolol (low standard) | 0.7 ± 0.9 | 4 |
| Antipyrine (high standard) | 17.4 ± 5.7 | 4 |
| Hydroxyzine | 355.89 ± 59.02 | 3 |
| PEG₅-Hydroxyzine | 131.60 ± 15.84 | 3 |
| PEG₇-Hydrozyine | 12.01 ± 2.97 | 3 |
| Cetirizine | 1.37 ± 0.37 | 3 |
| PEG₅-Cetirizine | 4.32 ± 0.26 | 3 |
| PEG₇-Cetirizine | 1.13 ± 0.05 | 3 |

### Reference Example 5

### Bioavailability Assay

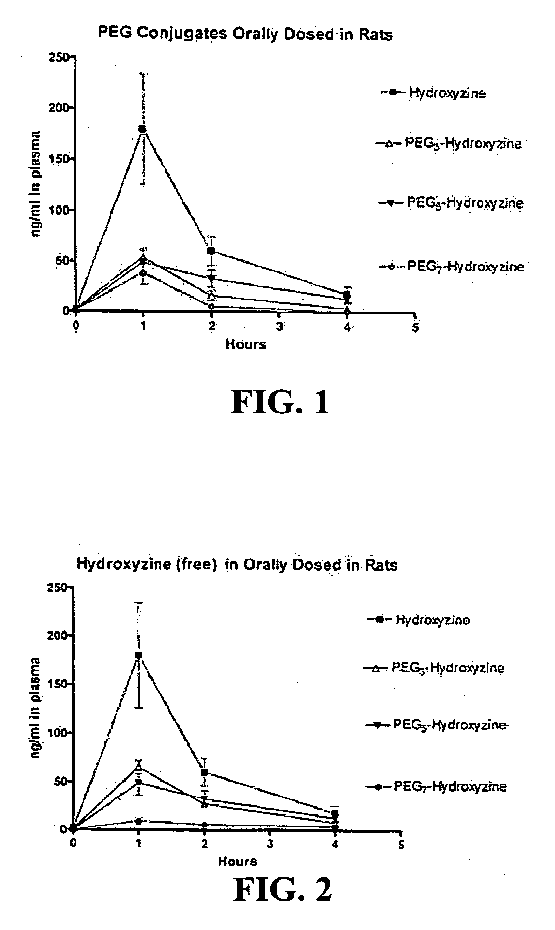

Several hydroxyzine conjugates were orally administered to rats followed by periodic testing of plasma to determine the amount of conjugate (or control) present in the plasma. LC-MS/MS was conducted with a Zorbax XDB C-8 column 2.1 x 50 mm, 1.8 µm particle size, 150 µL/min. Buffers used were: "A" as 0.1% formic acid, 20% acetonitrile; "B" 0.1 % formic acid, 70% acetonitrile; gradient elution was from 0% to 100% B in 2.5 minutes. Detection was accomplished with an MRM-Mass Spectrometer set at 389-201 m/z, 521-201 m/z, 609-201 m/z and 697-201 m/z corresponding to hydroxyzine, cetirizine, PEG₃-hydroxyzine, PEG₅-hydroxyzine, PEG₇-hydroxyzine. Results are presented in FIG. 1.

### Reference Example 6

### Free Drug Assay (Hydroxyzine)

Several hydroxyzine conjugates were orally administered to rats followed by periodic testing of plasma to determine the amount of free hydroxyzine (or control) present in the plasma. LC-MS/MS was conducted was described in Example 5. Results are presented in FIG. 2.

### Reference Example 7

### Free Drug Assay (Cetirizine)

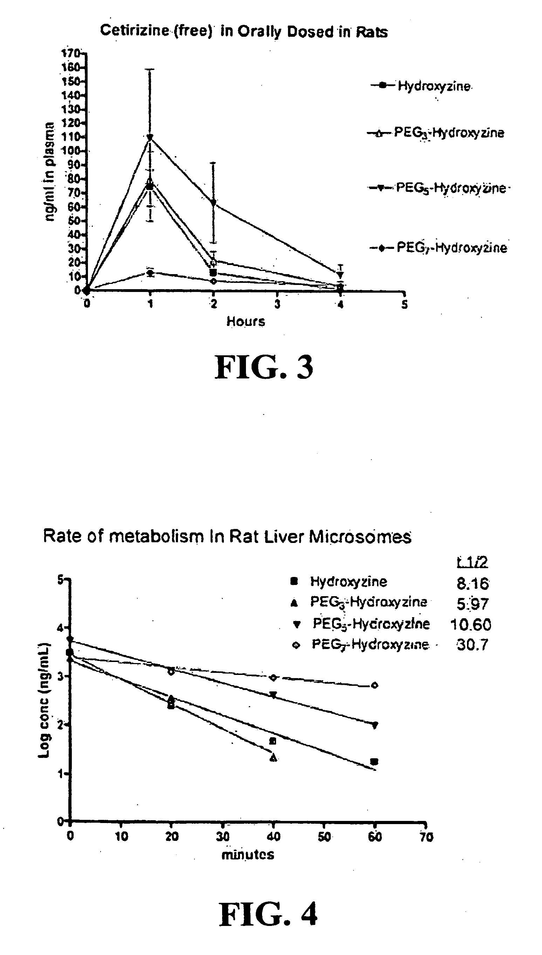

Several cetirizine conjugates were orally administered to rats followed by periodic testing of plasma to determine the amount of free cetirizine (or control) present in the plasma. LC-MS/MS was conducted as described in Example 5. Results are presented in FIG. 3.

### Reference Example 8

### Metabolism Assay - In Vitro

Several hydroxyzine conjugates combined with rat liver enzymes in vitro in the presence of an NADPH regenerating buffer. The conjugate (or control or interest) was added and incubated at 37°C. Phase I metabolism was assured inasmuch as PAPS and UDPGA (substances necessary for the sulfonation and glucuronidation Phase II reactions) were not added to the reaction system. Results are presented in FIG. 4.

### Reference Example 9

### Metabolism Assay - In Vivo

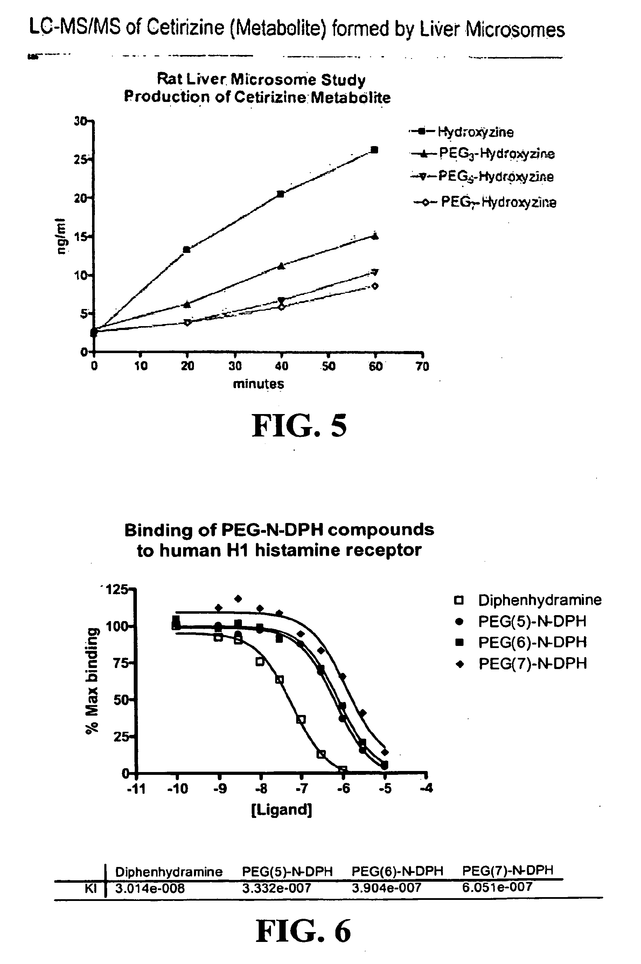

Sprague-Dawley female rats (16 rats total, 180g each) were dosed orally with: (1) hydroxyzine; (2) PEG₃-hydroxyzine; (3) PEG₅-hydroxyzine; and (4) PEG₇-hydroxyzine to give a hydroxyzine dose of 5 mg/kg and blood samples were taken at 1 hour, 2 hours, and 4 hours. Samples were centrifuged and plasma was collected and frozen at - 80 °C until ready for analysis. Results are presented in FIG. 5

### Example 10

### Receptor Binding at H₁ Histamine Receptors

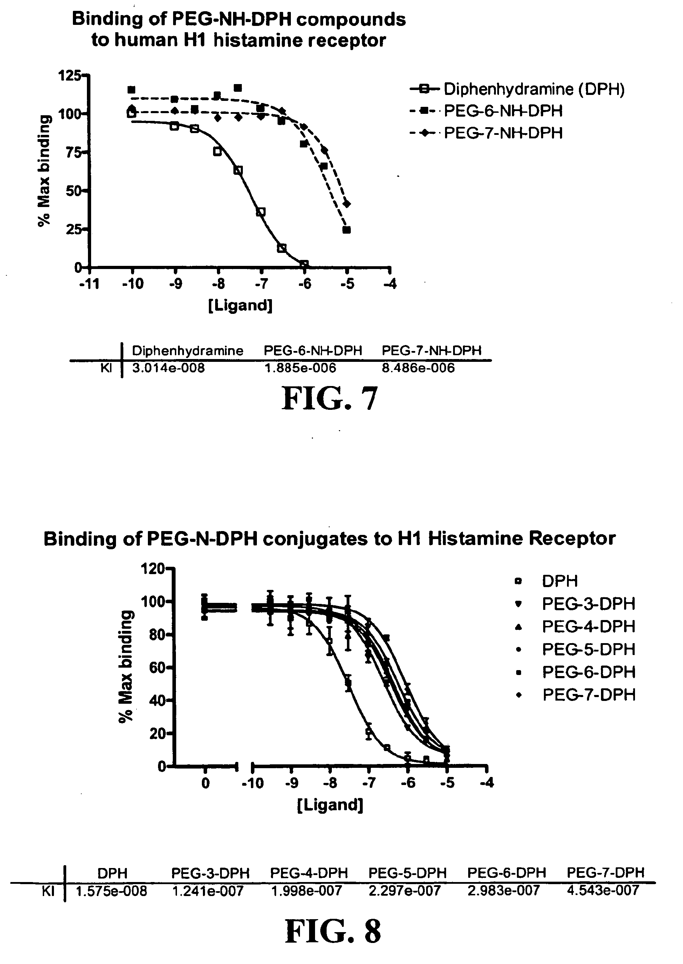

Receptor binding studies were performed using human recombinant H₁ histamine receptors in accordance with the procedures associated with Novascreen catalog # 100-0456. (Caliper Life Sciences, Hopkinton, MA). Competitive inhibition studies were employed using displacement of the high affinity ligand [³H]pyrilamine (Kd 1.0 nM) with non-specific binding determined using 10 µM triprolidine. This study was performed using duplicate samples per data point (n=2). The binding affinities (Ki) of various PEG-diphenhydramine conjugates are provided in Table III, below. The assay was repeated with various PEG-diphenhydramine conjugates and compounds tested in this repeated assay are marked with an asterisk in Table III. The relative binding affinities that were obtained for those compounds tested in both runs of the assay are in the same range. Additional results are provided in Figures 6 through 8.

**Table III**

| **Binding Affinities of Various PEG-Diphenhydramine Conjugates** | | | | |
|---|---|---|---|---|
| **Compound** | **PEG units** | **n number of samples run per data point** | **Ki (nM)** | **Fold vs parent** |
| Diphenhydramine HCl | 0 | 2 | 30.14 | 1 |
| Diphenhydramine HCl* | 0 | 3 | 15.8 | 1 |
| mPEG(3)-N-Diphenhydramine* | 3 | 3 | 124 | 7.8 |
| mPEG(4)-N-Diphenhydramine* | 4 | 3 | 200 | 12.7 |
| mPEG(5)-N-Diphenhydramine | 5 | 2 | 333.2 | 11.1 |
| mPEG(5)-N-Diphenhydramine* | 5 | 3 | 230 | 14.6 |
| mPEG(6)-N-Diphenhydramine | 6 | 2 | 390.4 | 13.0 |
| mPEG(6)-N-Diphenhydramine* | 6 | 3 | 298 | 18.9 |
| mPEG(7)-N-Diphenhydramine | 7 | 2 | 605.1 | 20.1 |
| mPEG(7)-N-Diphenhydramine* | 7 | 3 | 454 | 28.7 |
| mPEG(6)-NH-Diphenhydramine | 6 | 2 | 1885 | 62.5 |
| mPEG(7)-NH-Diphenhydramine | 7 | 2 | 8486 | 282 |

### Example 11

### Receptor Binding at H₁, H₂, M₁, M₂, and M₃ Receptors

To determine the binding affinity of PEG-diphenhydramine at other receptors, binding studies were performed using PEG-6-DPH[mPEG(6)-N-Diphenhydramine] at the H₁ histamine receptors, H₂ histamine receptors, M₁ muscarinic receptors, M₂ muscarinic receptors, and M₃ muscarinic receptors in accordance with the procedures associated with Novascreen catalog numbers 100-0456, 100-0086, 100-0038, 100-0039 and 100-0040, respectively (Caliper Life Sciences, Hopkinton, MA). Experiments were performed once per receptor (N=1) with triplicate samples per data point (n=3). Details of these experiments are shown in Table IV, below.

**Table IV**

| **Experimental Details of Screening-mode Receptor Binding Studies on PEG-6-DPH.** | | | | | |
|---|---|---|---|---|---|
| **Receptor** | **Species** | **Radiolabelled ligand** | **Concentration (radioligand)** | **Non-specific determinant** | **Positive control** |
| **H₁** | Human (recomb) | [³H]pyrilamine | 1 nM | Triprolidine 10 µM | Triprolidine |
| **H₂** | Guinea pig (striatal memb) | [¹²⁵I] aminopotentidine | 0.1 nM | Tiotidine 3 µM | Tiotidine |
| **M₁** | Human (recomb) | [³H]scopolamine N-methylchloride | 0.5 nM | (-) scopolamine methobromide 1 µM | (-) scopolamine methobromide |
| **M₂** | Human (recomb) | [³H]scopolamine N-methylchloride | 0.5M | (-) scopolamine methobromide 1 µM | (-) scopolamine methobromide |
| **M₃** | Human (recomb) | [³H]scopolamine N-methylchloride | 0.2 nM | (-) scopolamine methobromide 1 µM | (-) scopolamine methobromide |

The results of these experiments are summarized in Table V, below:

**Table V**

| **Binding Affinities (Ki) of PEG-6-DPH and DPH at the Histamine and Muscarinic Receptors, as Determined by Screening Experiments** | | | | | |
|---|---|---|---|---|---|
| | **Ki values (nM)** | | **DPH selectivity** | **PEG-6-DPH selectivity** | **Fold change** |
| **Receptor** | **DPH** | **PEG-6-DPH** | **Fold vs H₁** | **Fold vs H₁** | **DPH vs PEG-6-DPH** |
| **H₁ histamine** | **17.8** | **474** | 1.0 | 1.0 | 26.63 |
| **H₂ histamine** | 518 | No binding | - | - | - |
| **M1 (h)** | 549 | *2670** | - | - | - |
| **M2 (h)** | 587 | 1080 | 3.3.0 | 2.3 | 1.84 |
| **M3 (h)** | 776 | *1070 ** | - | - | - |

| | | | | | |
|---|---|---|---|---|---|
| **Values are extrapolated from incomplete curves, and are therefore very approximate.* | | | | | |

The results show that PEG-6-DPH retains high H₁ selectivity over H₂ - since no specific binding to H₂ receptors was observed. For muscarinic receptors, specific binding was observed at the M₂ subtype, which was close in affinity (low µM) to that of diphenhydramine. In view of the experiments, it is believed that the values for binding at H₁ and M₂ receptors are reliable. The values calculated for the H₂, M₁ and M₃, receptors, which are based on an experimental design that employed concentrations of ligand that were too low to generate full binding curves, are approximations.

### Example 12

### Metabolism

The metabolic stability of the PEG-diphenhydramine conjugates in human hepatocytes was determined by examining the amount of unmetabolized parent compound remaining following incubation with human hepatocytes. This experimental design accounts for the possibility that not all conjugates will be equally permeable across the cell membrane. The intracellular location of the metabolic enzymes requires that the drugs first enter the cell. Metabolic stability experiments using whole hepatocytes rather than microsomes include this parameter, and provide the most relevant system for understanding overall metabolic stability of these compounds.

The half-lies set forth in Table VI demonstrate that PEG conjugation does not slow the rate of metabolism of diphenhydramine in hepatocytes. Based on the initial rate of loss of the parent molecule (n=1), only relatively slight differences are noted between the compounds, with mPEG(5)-N-diphenhydramine demonstrating a moderately faster metabolic breakdown than diphenhydramine itself.

**Table VI**

| Metabolism of Diphenhydramine and PEG-Diphenhydramine Conjugates | |
|---|---|
| | **Half-Life (t_{1/2}) (hepatocytes)** |
| Diphenhydramine | 139.3 minutes |
| mPEG(5)-N-Diphenhydramine | 44.2 minutes |
| mPEG(6)-N-Diphenhydramine | 122.9 minutes |
| mPEG(7)-N-Diphenhydramine | 88.4 minutes |

It was noted that species differences in hepatocyte metabolism of diphenhydramine verses PEG(6)-N-Diphenhydramine were apparent. Both in humans and dog models, hepatic intrinsic clearance of PEG(6)-N-Diphenhydramine is faster than that of the parent diphenhydramine. In rat hepatocyte incubation, however, PEG(6)-N-Diphenhydramine shows slower metabolic breakdown than diphenhydramine.

### Example 13

### Protein Binding

Diphenhydramine has been shown to produce electrophysiologic effects via interaction with cardiac hERG channels in published *in vitro* studies. Hence, PEG(6)-N-Diphenhydramine and PEG(7)-N-Diphenhydramine conjugates were evaluated for their ability to interact with cloned hERG channels stably expressed in HEK cells. Inhibition of the hERG current was measured using electrophysiologic techniques and IC₅₀ values for the PEG-diphenhydramine conjugates were calculated from a concentration-response curve.

Diphenhydramine and PEG(6)-N-Diphenhydramine were equipotent inhibitors of the hERG channel with IC₅₀ values of 3.18 µM and 3.79 µM, respectively. The IC₅₀ of PEG(7)-N-Diphenhydramine was 4-fold lower (12.99 µM). These data suggest that the PEG-diphenhydramine conjugates retain the hERG channel inhibition properties of the parent diphenhydramine molecule.

### Example 14

### In Vitro Safety Pharmacology

The objective of this study was to determine the potential effects of escalating concentrations of conjugates on electrophysiological (PQ, QRS, RR, QT, QTc), and mechanical (dLVP/dtₘₐₓ and dLVP/dtₘᵢₙ) properties in spontaneously beating rat hearts. Measurements for all physiological parameters (PQ, QRS, RR, QT, dLVP/dtₘₐₓ, dLVP/dtₘᵢₙ) were made manually using EMKA ECG Auto software (QTc [Fridericia²] was calculated) from intervals of beats manifesting sinus rhythm. Means for all animals receiving the test article were calculated for all concentrations. Plots of mean values (±SEM) for all animals plus the negative control animals were made for all parameters versus concentration. Plots of the positive control were made for all parameters versus concentration. Plots of values of all parameters minus the values for vehicle were made with baseline adjustment.

The protocol responded to the positive control in a manner anticipated by the pharmacological properties of quinidine. PEG(5)-N-Diphenhydramine, like quinidine, manifested greater negative dromotropism (lengthening of PQ and QRS) than diphenhydramine. PEG(5)-N-Diphenhydramine produced greater cardiodeceleration at 10⁻⁵ M than the other two conjugates. PEG(5)-N-Diphenhydramine appeared to retard ventricular repolarization (QT and QTc) slightly more than diphenhydramine or quinidine. PEG(5)-N-Diphenhydramine exerted a striking positive inotropism and lusitropism at 10⁻⁵ M when compared to quinidine or diphenhydramine. PEG(5)-N-Diphenhydramine appeared to possess a vasodilatory effect on coronary smooth muscle not shown by either diphenhydramine or quinidine. It is important to further compare the electrophysiological effects on a protocol that possess ion channels more similar to man (e.g., guinea pig).

Guinea pig studies were performed with the objective of determining the potential effects of escalating concentrations conjugates. Measurements for all physiological parameters (PQ, QRS, RR, QT, dLVP/dtₘₐₓ, dLVP/dtₘᵢₙ) were made manually using EMKA ECG Auto software (QTc [Fridericia²] was calculated) from intervals of beats manifesting sinus rhythm. Means for all animals receiving the test article were calculated for all concentrations. Plots of mean values for all animals plus the negative control animals were made for all parameters versus concentration. Plots of the positive control were made for all parameters versus concentration. Plots of values of all parameters minus the values for vehicle were made with baseline adjustment. In addition, the effects of escalating concentrations compounds (10⁻⁸, 10⁻⁷, 10⁻⁶, 10⁻⁵ and 10⁻⁴ M) and vehicle (Krebs) on electrophysiological and mechanical parameters in guinea pig hearts were performed

Both rat and guinea pig preparations responded similarly. The validity of the test preparation to respond to changes in chronotropy, dromotropy, inotropy, and lusitropy is supported by the response to a compound of known pharmacology-quinidine. In general heart rate slowed, QRS and PQ durations increase only for 10⁻⁵ M concentration of PEG(5)-N-Diphenhydramine, QT but not QTc lengthened slightly for all test articles but for quinidine for which it lengthened dramatically, myocardial contractility and lusitropy both decreased in dose-response manners after both increasing only for the 10⁻⁸ M concentrations. Coronary perfusion either decreased slightly or not at all, except for PEG(5)-N-Diphenhydramine for which it increased dramatically. 10⁻⁴ M concentrations for all test articles were lethal. All changes may be attributed to effects of the test articles on specific ion channels regulating calcium (for chronotropy, inotropy, lusitropy, and dromotropy) and potassium kinetics (for chronotropy and ventricular repolarization).

### Example 15

### Peripheral Efficacy

Wheal and flare experiments

Wheal and flare (erythema) experiments were performed in rats in order to measure in *vivo* activity of PEG-diphenhydramine. Intradermal injection of histamine produces a "wheal and flare" response in the skin as a result of histamine-induced mast cell degranulation, which is prevented by treatment with effective antihistamines.

Diphenhydramine in doses 0.01, 0.03, 0.1 and 0.3 mg/kg (n=4 per condition), mPEG(6)-N-diphenhydramine in doses 0.03, 0.1 and 0.3 mg/kg (n=4 per condition), and a saline control (n=8) were used. All values represent "diphenhydramine equivalent" concentrations, i.e., molar equivalents.

The first animal was dosed with 1 mg/kg mPEG(6)-N-diphenhydramine, and suffered convulsions and died. The highest dose tested was then reduced to 0.3 mg/kg for all animals. The particulars of the dosing scheme are provided in Table VII, below.

**Table VII**

| Preparation of Test Articles | | | | |
|---|---|---|---|---|
| Final Dose (mg/kg) | Concentration to Use (mg/mL) | Injection Volume (uL) (375 g rat) | Volume to add per 5 mL | Of Solution |
| 0.03 | 0.09 | 125 | 0.5 | 1 mg/mL |
| 0.1 | 0.3 | 125 | 0.5 | 3 mg/mL |
| 0.3 | 0.9 | 125 | 1.50 | 3 mg/mL |
| 1 | 3 | 125 | As is | ---- |

Experimental details

Histamine was prepared from histamine diphosphate salt at 1 mg/mL (3.26 mM) in PBS, and was administered by intradermal injection 2.5 minutes after IV injection of the test article to the tail vein. At 5, 10, 20, 30 and 60 minutes post-injection, wheal sizes were measured using calipers and areas calculated (width x length, mm²). Flare responses were measured by eye, and were graded on a scoring system of 1 (least significant) - 5 (most severe).

Results: Flare Response

Visual scoring of flare response was performed to determine whether mPEG(6)-N-Diphenhydramine reduced the histamine effect. Whereas saline treatment produces a flare that has an average maximum at ten minutes, tailing off between 10-30 minutes, and gone by 60 minutes, diphenhydramine and mPEG(6)-N-diphenhydramine at the highest concentrations produce no detectable flare. (Note, some minimal response, or "noise," was observed for mPEG(6)-N-Diphenhydramine at the five minute time point). As the drug concentrations decrease, the protective effect begins to wane. Thus, at 0.03 mg/kg mPEG(6)-N-diphenhydramine, flare responses begin to return, while at the same concentration diphenhydramine still suppresses the response effectively. At 0.01 mg/kg diphenhydramine however, the protective effect of diphenhydramine begins to decrease, as flare responses are recorded.

These data suggest that both diphenhydramine and mPEG(6)-N-diphenhydramine suppress the wheal response, and that mPEG(6)-N-diphenhydramine is slightly less potent than diphenhydramine, since the antihistamine effect is lost at concentrations (0.03 mg/kg) where diphenhydramine is still effective.

Results: Wheal Response

At all concentrations tested, a wheal was generated which reached a plateau in size by approximately 30 minutes. Diphenhydramine produced a clear dose-dependent reduction in the size of this wheal. Higher doses of mPEG(6)-N-diphenhydramine (0.1, 0.3 mg/kg) reduced the size of the wheal compared with control injection, although the degree of this effect was less than that for the same diphenhydramine concentration. mPEG(6)-N-Diphenhydramine (0.03 mg/kg) did not produce a detectable change in wheal size.

When the dose-response effects from the 20 minute time-point are analyzed using "percent decrease in the wheal response," the effects show that diphenhydramine produces approximately 40% change in wheal size in the 0.1 - 0.3 mg/kg dose range. In this same dose range, mPEG(6)-N-diphenhydramine produces approximately 25% change in wheal response, and the shape of the curve suggests that this is not maximal.

Further study

A further study having the objective of comparing the antihistamine effects of diphenhydramine, PEG(5)-N-Diphenhydramine and PEG(7)-N-Diphenhydramine when administered via intravenous infusion was performed. Briefly, rats received a continuous infusion via the jugular vein catheter for 60 minutes. The infusion rate was changed to maintain a steady state over the last 20 minutes of the infusion (61 to 80 minutes). Sixty minutes after the start of the infusion period, the rats were given four (4) interdermal (ID) injections of histamine (200 mg/mL in saline; dose 50 µL at10 µg/rat). In addition, each rat was given an injection of Trypan Blue via the JVC (0.4% in saline, 0.5 mL volume). Twenty minutes after the histamine injections a caliper (Fowler Sylvac Ultra-Cal Mark III) was used to measure the wheal and flare (the diameter of the blue area). Two measurements of the diameter of the blue area at the site of histamine injection were made at 90 degrees from one another. An evaluation of the bulge of the wheal reaction was evaluated on a scale of 0, 1, and 2. Blood samples collected via cardiac puncture were used for diphenhydramine quantitation. The blood samples were placed in vacutainer tubes containing K2 EDTA. The rats were exsanguinated and the brain was rinsed in ice cold saline, wrapped in foil and frozen. The brain and the plasma samples were analyzed for drug content.

A dose dependent reduction in wheal size was observed for diphenhydramine at all concentrations of histamine tested. No significant change in wheal area is detected between 10 µg and 30 µg histamine. Thus, diphenhydramine provides a good dose-response in wheal and flare in rats, EC₅₀ is ∼ 400 ng/mL target plasma concentration (i.e., ∼ 1.6 mM) (based on data from days 1 and 3). PEG(5)-N-Diphenhydramine demonstrated efficacy, but toxicity occurs at same concentrations. PEG(5)-N-Diphenhydramine is ≥ 10-fold weaker than diphenhydramine based on efficacy at diphenhydramine-equivalent target plasma concentrations. PEG(7)-N-Diphenhydramine showed no efficacy in concentration range tested.

### Example 16

### Oral Bioavailability

Oral bioavailability was determined in rats by comparing the pharmacokinetic profile (plasma concentration vs time) following IV and oral dosing of four compounds: DPH (diphenhydramine); PEG-5-DPH [mPEG(5)-N-diphenhydramine]; PEG-6-DPH [mPEG(6)-N-diphenhydramine]; and PEG-7-DPH [mPEG(7)-N-Diphenhydramine]. For IV doses, 1 mg/kg of DPH-equivalent were used. For oral doses, 5mg/kg if DPH-equivalent were used.

Preparation of dosing solutions

Molar equivalent doses of all four compounds were administered, such that the final IV dose (1 mg/kg) was 1.03 µM (0.3 mg/mL DPH) and the final oral dose (5 mg/kg) was 5.14 µM (1.5 mg/mL DPH). Due to limited solubility of the PEG-DPH conjugates in the aqueous buffer, samples were prepared in 2% ethanol. The final dose ranged from 0.30 - 0.58 mg for IV administration, and from 1.50 - 2.90 mg for oral administration. Tables VIII and IX list the dosing parameters of the compounds.

**Table VIII**

| **Dosing Parameters of Tested Compounds** | | | | |
|---|---|---|---|---|
| Compound | Formula weight | Molar ratio | Concentration (mg/mL) | Adjusted Concentration (mg/mL) |
| Diphenhydramine | 291.8 | 1.00 | 3.00 | |
| mPEG(5)-N-Diphenhydramine | 475.6 | 1.63 | 4.89 | 2.44 |
| mPEG(6)-N-Diphenhydramine | 519.7 | 1.78 | 5.34 | 2.67 |
| mPEG(7)-N-Diphenhydramine | 563.7 | 1.93 | 5.80 | 2.70* |

| | | | | |
|---|---|---|---|---|
| *Note: 2.70 mg/mL was used in error (it should be 2.90 mg/mL) | | | | |

**Table IX**

| **Dosing Parameters of Tested Compounds** | | | | | | |
|---|---|---|---|---|---|---|
| Compound | Volume Used-IV (mL) | IV dose (mg) (*1 mg*/*kg)* | IV dose (moles) | Vol used - PO (mL) | Oral dose (mg) *(5 mg*/*kg)* | Oral dose (moles) |
| Diphenhydramine | 0.1 | 0.30 | 1.03E-06 | 0.5 | 1.50 | 5.14E-06 |
| mPEG(5)-N-Diphenhydramine | 0.2 | 0.49 | 1.03E-06 | 1.0 | 2.44 | 5.14E-06 |
| mPEG(6)-N-Diphenhydramine | 0.2 | 0.53 | 1.03E-06 | 1.0 | 2.67 | 5.14E-06 |
| mPEG(7)-N-Diphenhydramine | 0.2 | 0.54 | 9.58E-07 | 1.0 | 2.70 | 4.79E-06 |

Experimental details

Animals were dosed in triplicate by bolus injection to the femoral vein (IV) or oral gavage (PO), and whole blood was collected from the carotid artery from all animals at the appropriate time points for preparation of plasma samples. Quantitation of test article in plasma was performed by LC-MS/MS.

Plasma concentration-time profiles following IV and oral administration are displayed in Figures 14 and 15. The corresponding pharmacokinetic parameters are shown in Tables X and XI,below.

Results: IV administration

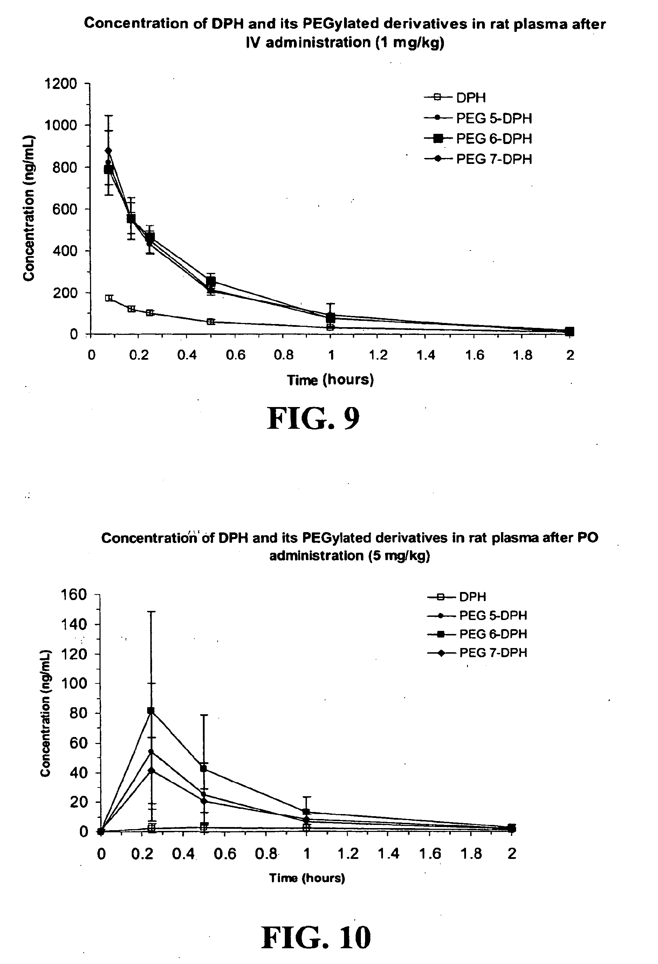

The pharmacokinetic parameters of PEG-N-DPH conjugates following IV administration in rats are provided in Table X. Plasma concentration-time profiles of DPH and PEG-N-DPH conjugates following IV administration in rats is provided in FIG. 9.

**Table X**

| **Pharmacokinetic Parameters of PEGN-DPH Conjugates Following IV Administration in Rats** | | | | | |
|---|---|---|---|---|---|
| | Cmax | AUCinf | CL | Vss | T1/2 |
| Compound | ng/mL | hr*ng/mL | mL/hr/kg | mL/kg | hr |
| Diphenhydramine | 172 | 118 | 8460 | 7002 | 0.71 |
| mPEG(5)-N-Diphenhydramine | 821 | 391 | 4172 | 1977 | 0.33 |
| mPEG(6)-N-Diphenhydramine | 790 | 405 | 4399 | 1910 | 0.38 |
| mPEG(7)-N-Diphenhydramine | 882 | 410 | 4705 | 2074 | 0.43 |

The most notable feature of the pharmacokinetic profile following IV administration in rats is the reduced rate of clearance (CL) and lower volume of distribution (Vss) of the PEG-DPH conjugates compared with the parent DPH. Clearance rates show a 2-fold reduction, and volume of distribution values are 4-fold lower for the PEG-DPH conjugates compared with the parent diphenhydramine compound, resulting in higher concentrations remaining in the plasma for a longer time period.

Results: Oral administration

The pharmacokinetic parameters of PEG-N-DPH conjugates following oral administration in rats are provided in Table XI. Plasma concentration-time profiles of DPH and PEG-N-DPH conjugates following oral administration in rats is provided in FIG. 10.

**Table XI**

| **Pharmacokinetic Parameters of PEG-N-DPH Conjugates Following.Oral Administration in Rats** | | | | | |
|---|---|---|---|---|---|
| | Cmax | Tmax | AUCinf | T1/2 | F |
| Compound | ng/mL | hr | hr*ng/mL | hr | % |
| Diphenhydramine | 2.9 | 0.5 | 5.7 | 1.1 | 1.0 |
| mPEG(5)-N-Diphenhydramine | 53.6 | 0.25 | 29.4 | 0.38 | 1.5 |
| mPEG(6)-N-Diphenhydramine | 81.9 | 0.25 | 49.1 | 0.39 | 2.4 |
| mPEG(7)-N-Diphenhydramine | 41.3 | 0.25 | 26.6 | 0.47 | 1.3 |

Oral bioavailability in rats was extremely low for all four compounds, indicating that oral dosing in rodents will not be practical (although some increase in bioavailability was observed for the PEG-N-DPH conjugates compared with DPH). Since human oral bioavailability is approximately 72% for diphenhydramine, this suggests that rodents provide only a limited model for pharmacokinetic behavior of these conjugates in humans and may not be reliably predictive.

### Example 17

### Blood-Brain Barrier ("BBB") Penetration

The effect on brain distribution of DPH (diphenhydramine) following conjugation to PEG was determined by comparing the brain:plasma concentration ratios of four compounds: DPH (diphenhydramine); PEG-5-DPH [mPEG(5)-N-diphenhydramine]; PEG-6-DPH [mPEG(6)-N-diphenhydramine]; and PEG-7-DPH [mPEG(7)-N-diphenhydramine]. The four compounds were tested following intravenous injection to the tail vein in rats. PEG-DPH solutions were prepared in 2% ethanol (as discussed previously), at the concentrations shown in Table XII, below.

**Table XII**

| **Dosing Solutions for Brain:Plasma Experiment in Rats** | | | | | |
|---|---|---|---|---|---|
| Compound | Volume Used-IV (mL) | IV dose (mg) | IV dose (moles) | Concn (mg/mL) | Adjusted concn |
| Diphenhydramine | 0.3 | 0.90 | 3.08E-06 | 3 | |
| mPEG(5)-N-Diphenhydramine | 0.3 | 0.73 | 1.54E-06 | 4.89 | 2.44 |
| mPEG(6)-N-Diphenhydramine | 0.3 | 0.80 | 1.54E-06 | 5.34 | 2.67 |
| mPEG(7)-N-Diphenhydramine | 0.3 | 0.81 | 1.44E-06 | 5.80 | *2.70** |

| | | | | | |
|---|---|---|---|---|---|
| * should be 2.90 | | | | | |

As the diphenhydramine molar dose was twice that of the PEG-DPH conjugates, the compounds were therefore not administered in equimolar quantities. This fact has been taken into account in the following calculations.

After ensuring a single rat remained viable following a test dose, the rats (4 per condition) were dosed by tail vein injection and were sacrificed one hour later. Blood was collected by cardiac puncture, and brain tissue was collected. Plasma was prepared and brain tissue homogenized to enable quantitation of drug content by LC-MS/MS. Concentrations of each compound were determined and brain:plasma ratios calculated accordingly.

Results

The results demonstrate a marked reduction in brain:plasma ratios for the PEG-DPH derivatives compared with diphenhydramine. Diphenhydramine displays a brain:plasma ratio of 21:1, which lies in good agreement with that reported by others. This suggests that at the chosen one hour time point for sacrifice, diphenhydramine had reached an equilibrium distribution across all tissues following the tail vein injection.

By contrast with diphenhydramine, all 3 PEG-DPH conjugates display a brain:plasma ration of 0.2:1, i.e., a five-fold lower concentration in the brain than in the plasma.

Relative to diphenhydramine, therefore, the PEG-DPH conjugates have 105-fold lower brain penetration, suggesting a significant degree of CNS exclusion. Quantitation of diphenhydramine and PEG-DPH conjugates in brain and plasma are provided in Table XIII.

**Table XIII**

| Quantitation of DPH and PEG-DPH in Brain and Plasma Following Tail Vein Injection in Rats | | | | |
|---|---|---|---|---|
| Compound | Brain concentration (ng/g) | Fold change (vs DPH)* | Plasma concentration (ng/mL) | Brain:plasma ratio |
| Diphenhydramine | 5460.7 ± 1137.9 | 1 | 258.0 ± 59.1 | 21 to 1 |
| PEG-5-DPH | 97.9 ± 32.9 | 28 X | 415.3 ± 78.7 | 0.2 to 1 |
| PEG-6-DPH | 45.1 ± 4.4 | 60.5 X | 237.0 ± 41.1 | 0.2 to 1 |
| PEG-7-DPH | 106.5 ± 27.1 | 26 X | 544.8 ± 57.9 | 0.2 to 1 |

| | | | | |
|---|---|---|---|---|
| *NOTE: DPH molar dose is twice that of PEG-DPH conjugates. Relative change is therefore adjusted to reflect this. | | | | |

The pharmacokinetic experiments demonstrate that PEG conjugation produces a significant change in the biodistribution of diphenhydramine. Most notably, a dramatic exclusion from the brain is observed, with a 100-fold lower brain penetration than for the parent molecule. In addition, the PEG conjugates display a 3-4 fold lower volume of distribution, suggesting they undergo less tissue distribution and are relatively more concentrated in the plasma compartment.

## Claims

1. A compound having the following structure: 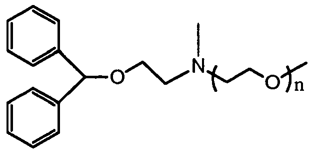 wherein:
n is an integer from 1 to 30.

2. The compound of claim 1, wherein n is an integer from 1 to 10.

3. The compound of claim 2, wherein n is an integer selected from 3, 4, 5, 6, and 7.

4. A composition comprising a compound of claim 1, and optionally, a pharmaceutically acceptable excipient.

5. A composition of matter comprising a compound of claim 1, wherein the compound is present in a dosage form.

6. Use of a compound of claim 1 in the manufacture of a medicament for binding histamine receptors.

## Patentansprüche

1. Verbindung mit der folgenden Struktur: 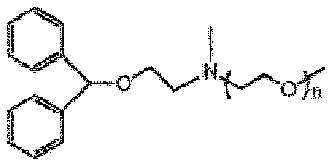 worin:
n eine ganze Zahl von 1 bis 30 bedeutet.

2. Verbindung nach Anspruch 1, worin n eine ganze Zahl von 1 bis 10 bedeutet.

3. Verbindung nach Anspruch 2, worin n eine ganze Zahl ist, die unter 3, 4, 5, 6 und 7 ausgewählt ist.

4. Zusammensetzung, die eine Verbindung nach Anspruch 1 und optional einen pharmazeutisch akzeptablen Träger enthält.

5. Zusammensetzung eines Gegenstandes, die eine Verbindung nach Anspruch 1 enthält, wobei die Verbindung in einer Darreichungsform vorliegt.

6. Verwendung einer Verbindung nach Anspruch 1 für die Herstellung eines Arzneimittels zum Binden an Histamin-Rezeptoren.

## Revendications

1. Composé ayant la structure suivante : 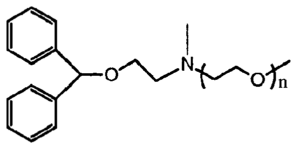 dans laquelle :
n représente un nombre entier de 1 à 30.

2. Composé suivant la revendication 1, dans lequel n représente un nombre entier de 1 à 10.

3. Composé suivant la revendication 2, dans lequel n représente un nombre entier choisi entre 3, 4, 5, 6 et 7.

4. Composition comprenant un composé de la revendication 1 et, éventuellement, un excipient pharmaceutiquement acceptable.

5. Composition comprenant un composé de la revendication 1, dans lequel le composé est présent sous une forme posologique.

6. Utilisation d'un composé de la revendication 1 dans la production d'un médicament pour la liaison aux récepteurs d'histamine.
